(19) Europäisches Patentamt
European Patent Office
Office européen des brevets

(11) EP 1 539 666 B1

(12) **EUROPÄISCHE PATENTSCHRIFT**

(45) Veröffentlichungstag und Bekanntmachung des Hinweises auf die Patenterteilung:
**07.01.2009 Patentblatt 2009/02**

(21) Anmeldenummer: **03748014.2**

(22) Anmeldetag: **12.09.2003**

(51) Int Cl.:
***C07C 45/50*** *(2006.01)* ***C07C 47/21*** *(2006.01)*
***C07C 47/12*** *(2006.01)* ***C07F 9/6558*** *(2006.01)*

(86) Internationale Anmeldenummer:
**PCT/EP2003/010166**

(87) Internationale Veröffentlichungsnummer:
**WO 2004/026803 (01.04.2004 Gazette 2004/14)**

(54) **VERFAHREN ZUR HERSTELLUNG VON DIALDEHYDEN UND/ODER ETHYLENISCH UNGESÄTTIGTEN MONOALDEHYDEN DURCH HYDROFORMYLIERUNG ETHYLENISCH UNGESÄTTIGTER VERBINDUNGEN**

METHOD FOR PRODUCING DIALDEHYDES AND/OR ETHYLENICALLY UNSATURATED MONOALDEHYDES BY HYDROFORMYLATING ETHYLENICALLY UNSATURATED COMPOUNDS

PROCEDE DE PRODUCTION DE DIALDEHYDES ET/OU DE MONOALDEHYDES ETHYLENIQUEMENT INSATURES PAR HYDROFORMYLATION DE COMPOSES ETHYLENIQUEMENT INSATURES

(84) Benannte Vertragsstaaten:
**AT BE BG CH CY CZ DE DK EE ES FI FR GB GR HU IE IT LI LU MC NL PT RO SE SI SK TR**

(30) Priorität: **13.09.2002 DE 10242636**

(43) Veröffentlichungstag der Anmeldung:
**15.06.2005 Patentblatt 2005/24**

(73) Patentinhaber: **BASF SE**
**67056 Ludwigshafen (DE)**

(72) Erfinder:
- **VOLLAND, Martin**
**69115 Heidelberg (DE)**
- **AHLERS, Wolfgang**
**67549 Worms (DE)**
- **EBEL, Klaus**
**68623 Lampertheim (DE)**
- **PACIELLO, Rocco**
**67098 Bad Dürkheim (DE)**
- **RÖPER, Michael**
**67157 Wachenheim (DE)**
- **MACKEWITZ, Thomas**
**68219 Mannheim (DE)**
- **BÖHM, Volker**
**67227 Frankenthal (DE)**
- **SAVA, Xavier**
**68161 Mannheim (DE)**
- **LÖBER, Oliver**
**55234 Freimersheim (DE)**
- **BEY, Oliver**
**67150 Niederkirchen (DE)**
- **STEPHAN, Jürgen**
**68163 Mannheim (DE)**
- **HAESE, Frank**
**67245 Lambsheim (DE)**

(74) Vertreter: **Reitstötter - Kinzebach**
**Ludwigsplatz 4**
**67059 Ludwigshafen (DE)**

(56) Entgegenhaltungen:
**WO-A-01/58589** **WO-A-02/083695**
**WO-A-03/018192** **WO-A-03/062251**
**WO-A-03/066642** **DE-A- 10 239 134**
**US-A- 5 710 344**

EP 1 539 666 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung von Dialdehyden und/oder ethylenisch ungesättigten Monoaldehyden durch Hydroformylierung wenigstens einer Verbindung mit mindestens zwei ethylenisch ungesättigten Doppelbindungen in Gegenwart eines Katalysators, der wenigstens einen Komplex eines Metalls der VIII. Nebengruppe mit wenigstens einem Pnicogenliganden umfasst.

Ethylenisch ungesättigte Monoaldehyde ("Enale") und Dialdehyde sind großtechnisch wichtige Zwischenprodukte. So lässt sich die Aldehyd-Gruppe leicht in eine Vielzahl anderer funktioneller Gruppen wie Amino-, Hydroxy-, Carboxygruppe etc. überführen. Ausgehend von Enalen erhält man so eine Vielzahl von auf anderen Synthesewegen schwer zugänglichen Verbindungen, die sich als bifunktionelle Synthesebausteine für Folgeumsetzungen eignen. Dialdehyde und die aus ihnen erhältlichen Diole, Diamine und Dicarbonsäuren eignen sich für eine Vielzahl von Anwendungen, z. B. zur Herstellung von Polyestern und Polyamiden sowie als Vernetzungsmittel für Polymere.

Es ist prinzipiell bekannt, Enale und Dialdehyde durch Hydroformylierung (Oxo-Synthese) von Verbindungen mit mindestens zwei ethylenisch ungesättigten Doppelbindungen herzustellen. Dabei werden die ethylenisch ungesättigten Verbindungen mit Kohlenmonoxid und Wasserstoff (Synthesegas) in Gegenwart eines Hydroformylierungskatalysators umgesetzt. Bei der Hydroformylierung von mehrfach ungesättigten Verbindungen kann es je nach Ort der Anlagerung der CO-Moleküle an die Doppelbindungen zur Bildung von Gemischen isomerer Aldehyde kommen. Zusätzlich kann es bei der Hydroformylierung von Diolefinen mit mehr als 4 Kohlenstoffatomen bzw. von drei- und mehrfach ungesättigten Verbindungen zu einer Doppelbindungsisomerisierung kommen. Aufgrund der großen technischen Bedeutung linearer ethylenisch ungesättigter Monaldehyde, insbesondere mit endständiger Doppelbindung und Aldehydfunktion ($\alpha,\omega$-Enale) sowie von linearen Dialdehyden ($\alpha,\omega$-Dialdehyden) besteht ein Bedarf an Hydroformylierungskatalysatoren, die ausgehend von $\alpha,\omega$-ethylenisch ungesättigten Verbindungen (wie $\alpha,\omega$-Dioelfinen) hohe Ausbeuten an $\alpha,\omega$-Enalen und/oder $\alpha,\omega$-Dialdehyden liefern. Derartige Katalysatoren werden im Rahmen dieser Erfindung als Katalysatoren mit hoher Selektivität bezeichnet.

Eine weitere Forderung, die an Hydroformylierungskatalysatoren gestellt wird, ist eine gute Stabilität, sowohl unter den Hydroformylierungsbedingungen als auch bei der Aufarbeitung, da Katalysatorverluste sich in hohem Maße negativ auf die Wirtschaftlichkeit des Verfahrens auswirken. Des Weiteren sollen Katalysatoren für die Hydroformylierung mehrfach ethylenisch ungesättigter Verbindungen sich durch eine hohe Aktivität bei möglichst geringen Temperaturen und möglichst geringen Reaktionsdrücken auszeichnen, um unerwünschte Nebenreaktionen, wie z. B. die Aldolreaktion, zu vermeiden.

WO 95/30680 und van Leeuwen et al., Organometallics 14, 3081 (1995) beschreiben Chelatphosphine mit Xanthen-Rückgrat, deren Anwendung bei der Rhodium-katalysierten Hydroformylierung endständiger Olefine zu hohen n-Selektivitäten führt. Bei den zur Hydroformylierung geeigneten ungesättigten Verbindungen werden auch Diolefine aufgeführt.

Van der Slot et al., Organometallics 19, 2504 (2000) beschreiben die Synthese von Phosphordiamid-Chelatliganden mit Bisphenol-oder Xanthen-Rückgrat, deren Diamid-Einheit durch Biuret-Gruppen gebildet wird, sowie die katalytischen Eigenschaften der Rhodium-Komplexe dieser Verbindungen bei der Hydroformylierung.

Die WO 00/56451 betrifft am Phosphoratom unter anderem mit Pyrrolderivaten substituierte, cyclische Oxaphosphorine und die Verwendung dieser als Liganden in Katalysatoren zur Hydroformylierung.

Die WO 01/58589 beschreibt Verbindungen des Phosphors, Arsens und des Antimons, basierend auf Diarylanellierten Bicyclo[2.2.2]-Grundkörpern und Katalysatoren, die diese als Liganden enthalten.

Die DE-A-100 23 471 beschreibt ein Verfahren zur Hydroformylierung unter Einsatz eines Hydroformylierungskatalysators, der wenigstens einen Phosphinliganden umfasst, der zwei Triarylphosphingruppen aufweist, wobei jeweils ein Arylrest der beiden Triarylphosphingruppen über eine Einfachbindung an eine nichtaromatische 5- bis 8-gliedrige carbocyclische oder heterocyclische verbrückende Gruppe gebunden ist. Dabei können die Phosphoratome als weitere Substituenten unter anderem auch Hetarylgruppen aufweisen.

Die DE-A-100 46 026 beschreibt ein Hydroformylierungsverfahren, bei dem man als Katalysator einen Komplex auf Basis einer Phosphor-, Arsen- oder Antimon-haltigen Verbindung als Liganden einsetzt, wobei diese Verbindung jeweils zwei ein P-, As- oder Sb-Atom und wenigstens zwei weitere Heteroatome aufweisende Gruppen gebunden an ein Xanthen-artiges Molekülgerüst aufweist.

US-A 5,710,344 betrifft die Hydroformylierung von Olefinen mittels Rhodiumkatalysatoren, die mit Chelatphosphordiamidit-Liganden mit Bisphenol- oder Bisnaphthol-Rückgrat und deren Phosphoratome unsubstituierte Pyrrolyl-, Imidazolyl- oder Indolylgruppen tragen können, modifiziert sind. Als Olefin wird u. a. 1,3-Butadien eingesetzt.

A. M. Trzeciak und J. J. Ziolkowski beschreiben in J. Organomet. Chem., 464 (1994), 107 - 111 die selektive Hydroformylierung von 1,5-Hexadien und 1,7-Octadien in Gegenwart der Katalysatorsysteme $Rh(acac)(P(OC_6H_5)_3)_2/P(OC_6H_5)_3$ beziehungsweise $Rh(acac)(CO)(P(C_6H_5)_3)/P(C_6H_5)_3$. Dabei wird die Bildung einer Vielzahl isomerer Mono- und Dialdehyde beobachtet. Die n-Selektivität dieser Katalysatorsysteme ist zur gezielten Herstellung von $\alpha,\omega$-Enalen bzw.-Dialdehyden unzureichend.

C. Botteghi et al. beschreiben in J. Mol. Catal. A: Chem 2001, 175, 17-25 die Herstellung von langkettigen linearen Dialdehyden durch Hydroformylierung von $\alpha,\omega$-Dienen oder $\omega$-Vinylaldehydacetalen. Die Selektivität bezüglich linearer $\alpha,\omega$-Dialdehyde ist bei Verwendung von Standard-Katalysatorsystemen wie $Rh(CO)_2(acac)$, $Rh(CO)_2(acac)P(C_6H_5)_3$ oder $Rh(CO)_2(acac)P(OC_6H_5)_3$ gering. Bei Einsatz eines in situ aus $RhH(CO)(P(C_6H_5)_3)_3$/Xantphos gebildeten Komplexes als katalytischen Prekursors erzielt man einen hohen $\alpha,\omega$-Diolefin-Umsatz und einen hohen Anteil an linearem Dialdehyd. Nachteilig an diesem Katalysatorsystem sind die sehr langen Reaktionszeiten und die hohen erforderlichen Katalysatoreinsatzmengen.

Die WO 03/018192 beschreibt ein Hydroformylierungsverfahren unter Einsatz eines Katalysatorkomplexes, der als Liganden wenigstens eine Pyrrolphosphorverbindung aufweist, bei der eine substituierte und/oder in ein anelliertes Ringsystem intergrierte Pyrrolgruppe über ihr pyrrolisches Stickstoffatom kovalent mit dem Phosphoratom verknüpft ist.

Die unveröffentlichte internationale Anmeldung PCT/EP 03/01245 beschreibt Phosphorchelatverbindungen, bei denen an die beiden Phosphoratome jeweils drei Stickstoffatome kovalent gebunden sind, die selbst Teil eines aromatischen Ringsystems sind, und deren Einsatz als Liganden für Hydroformylierungskatalysatoren.

Die WO 02/083695 beschreibt u. A. ein Verfahren zur Hydroformylierung von Olefinen unter Einsatz eines Hydroformylierungskatalysators, der einen Komplex eines Metalls der VIII. Nebengruppe mit mindestens einer Pnicogenchelatverbindung als Liganden umfasst. Diese Liganden weisen zwei Pnicogenatome enthaltende Gruppen auf, welche über ein Xanthen-artiges oder Triptycen-artiges Molekülgerüst miteinander verbunden sind und wobei an jedes Pnicogenatom mindestens eine Pyrrolgruppe über deren Stickstoffatom kovalent gebunden ist. Als zur Hydroformylierung geeignete Olefine werden neben einer Vielzahl weiterer auch ganz allgemein Di-oder Polyene genannt. Ausführungsbeispiele mit diesen Olefine sind nicht enthalten.

Der vorliegenden Erfindung liegt die Aufgabe zugrunde, ein Verfahren zur Hydroformylierung von Verbindungen mit mindestens zwei ethylenisch ungesättigten Doppelbindungen unter möglichst milden Druck- und/oder Temperaturbedingungen bei kurzen Reaktionszeiten und/oder Katalysatorbeladungen zur Verfügung zu stellen. Dabei soll bei der Hydroformylierung von Diolefinen vorzugsweise ein möglichst hoher Anteil an $\alpha,\omega$-Dialdehyden und/oder $\alpha,\omega$-Enalen bei gutem Umsatz erzielt werden (hohe n-Selektivität). Die Isomerisierung von im molekülenthaltenen ethylenisch ungesättigten Doppelbindungen soll möglichst vermieden werden. Insbesondere sollen auch die Katalysatorstandzeiten hoch sein.

Überraschenderweise wurde nun gefunden, dass diese Aufgabe durch ein Verfahren zur Hydroformylierung gelöst wird, wobei man als Hydroformylierungskatalysator wenigstens einen Komplex eines Metalls der VIII. Nebengrupe mit mindestens einem Pnicogenchelatliganden einsetzt, der zwei Pnicogenatome enthaltende Gruppen aufweist, welche über ein Xanthen-artiges oder Triptycen-artiges Molekülgerüst miteinander verbunden sind und wobei an jedes Pnicogenatom mindestens eine Pyrrolgruppe über deren Stickstoffatom kovalent gebunden ist.

Gegenstand der Erfindung ist daher ein Verfahren zur Herstellung von Dialdehyden und/oder ethylenisch ungesättigten Monoaldehyden durch Umsetzung (von 1.9-Decadion) mit Kohlenmonoxid und Wasserstoff in Gegenwart eines Hydroformylierungskatalysators, der wenigstens einen Komplex eines Metalls der VIII. Nebengruppe mit wenigstens einem Liganden umfasst, der ausgewählt ist unter Pnicogenchelatverbindungen der allgemeinen Formel I,

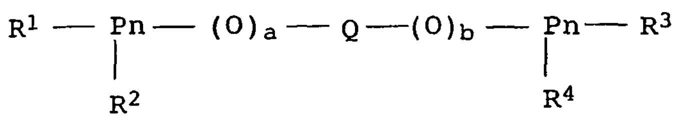

I

worin

Q eine Brückengruppe der Formel

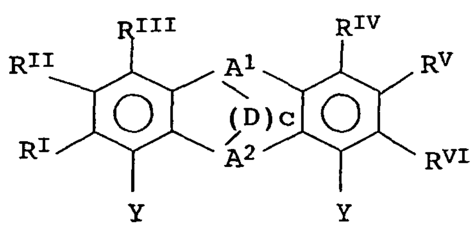

ist,
worin

$A^1$ und $A^2$ unabhängig voneinander für O, S, $SiR^aR^b$, $NR^c$ oder $CR^dR^e$ stehen, wobei

$R^a$,$R^b$ und $R^c$ unabhängig voneinander für Wasserstoff, Alkyl, Cyclo- alkyl, Heterocycloalkyl, Aryl oder Hetaryl stehen,

$R^d$ und $R^e$ unabhängig voneinander für Wasserstoff, Alkyl, Cyclo- alkyl, Heterocycloalkyl, Aryl oder Hetaryl stehen oder die Gruppe $R^d$ gemeinsam mit einer weiteren Gruppe $R^d$ oder die Gruppe $R^e$ gemeinsam mit einer weiteren Gruppe $R^e$ eine intramo- lekulare Brückengruppe D bilden,

D eine zweibindige Brückengruppe, ausgewählt aus den Gruppen

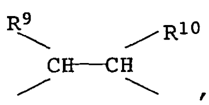
,
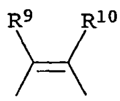
,
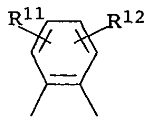
,
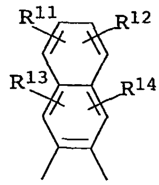

ist, in denen

$R^9$ und $R^{10}$ unabhängig voneinander für Wasserstoff, Alkyl, Cyclo- alkyl, Aryl, Halogen, Trifluormethyl, Carboxyl, Carboxylat oder Cyano stehen oder miteinander zu einer $C_3$- bis $C_4$-Alkylenbrücke verbunden sind,

$R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carb- oxylat, Cyano, Alkoxy, $SO_3H$, Sulfonat, $NE^1E^2$, Alky- len-$NE^1E^2E^{3+}X^-$, Acyl oder Nitro stehen,

c 0 oder 1 ist, (wenn c für 0 steht, liegt keine direkte Bin- dung zwischen $A^1$ und $A^2$ vor)

Y eine chemische Bindung darstellt,

$R^I$, $R^{II}$, $R^{III}$, $R^{IV}$, $R^V$ und $R^{VI}$ unabhängig voneinander für Wasser- stoff, Alkyl, Cycloalkyl, Heterocycloalkyl, Aryl, Hetaryl, $COOR^f$, COO-$M^+$, $SO_3R^f$, $SO^-_3M^+$, $NE^1E^2$, $NE^1E^2E^{3+}X^-$, Alky- len-$NE^1E^2E^{3+}X^-$, $OR^f$ , $SR^f$ , $(CHR^gCH_2O)_xR^f$, $(CH_2N(E^1))_xR^f$, $(CH_2CH_2N(E^1))_xR^f$, Halogen, Trifluormethyl, Nitro, Acyl oder Cyano stehen,
worin
$R^f$, $E^1$, $E^2$ und $E^3$ jeweils gleiche oder verschiedene Reste, ausgewählt unter Wasserstoff, Alkyl, Cycloalkyl oder Aryl bedeuten,

$R^g$ für Wasserstoff, Methyl oder Ethyl steht,

$M^+$ für ein Kation steht,

$X^-$ für ein Anion steht, und

x für eine ganze Zahl von 1 bis 120 steht,
oder
zwei benachbarte Reste, ausgewählt unter $R^I$, $R^{II}$, $R^{III}$, $R^{IV}$, $R^V$ und $R^{VI}$ zusammen mit zwei benachbarten Kohlenstoffatomen des Benzolkerns, an den sie gebunden sind, für ein kondensiertes Ringsystem, mit 1, 2 oder 3 weiteren Ringen stehen,

a und b — unabhängig voneinander die Zahl 0 oder 1 bedeuten

Pn — für ein Pnicogenatom ausgewählt aus den Elementen Phosphor, Arsen oder Antimon steht, und

$R^1$, $R^2$, $R^3$, $R^4$ — unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine $NE^1E^2$-Gruppe stehen, mit der Maßgabe, dass $R^1$ und $R^3$ über das Stickstoff- atom an das Pnicogenatom Pn gebundene Pyrrolgruppen sind oder worin $R^1$ gemeinsam mit $R^2$ und/oder $R^3$ gemeinsam mit $R^4$ eine zweibindige Gruppe E der Formel,

Py-I-W

bildet, worin

Py — eine Pyrrolgruppe ist, die über das pyrrolische Stick- stoffatom an das Pnicogenatom Pn gebunden ist,

I — für eine chemische Bindung oder für O, S, $SiR^aR^b$, $NR^c$, gegebenenfalls substituiertes $C_1$-$C_{10}$-Alkylen oder $CR^hR^i$ steht,

W — für Cycloalkyl, Cycloalkoxy, Aryl, Aryloxy, Hetaryl oder Hetaryloxy steht,

und

$R^h$ und $R^i$ oder eine dene bildet. — unabhängig voneinander für Wasserstoff, Alkyl, Cycloalkyl, Heterocycloalkyl, Aryl oder Hetaryl stehen, worin $R^1$ gemeinsam mit $R^2$ und/oder $R^3$ gemeinsam mit $R^4$ über die Stickstoffatome an das Pnicogenatom Pn gebun- Bispyrrolgruppe der Formel Py-I-Py

In einer speziellen Ausführungsform betrifft die vorliegende Erfindung ein Verfahren zur Hydroformylierung von Verbindungen, die wenigstens zwei ethylenisch ungesättigte Doppelbindung enthalten, unter Isolierung der gebildeten ungesätttigten Monoaldehyde.

Für den Zweck der Erläuterung der vorliegenden Erfindung versteht man unter dem Ausdruck "Anteil an linearem Dialdehyd" (beide Doppelbindungen endständig hydroformyliert) den Anteil an gebildetem n,n-Dialdehyd bezogen auf die Summe der gebildeten n,n-, n,iso-und iso,iso-Dialdehyde. Der n-Anteil berechnet sich somit nach der folgenden Gleichung:

$$\text{n-Anteil} = \frac{\text{Anteil an gebildetem n,n-Dialdehyd}}{\text{Summe aller gebildeten Dialdehyde}}$$

Summe aller Dialdehyde = Anteil an n,n-Dialdehyd +
Anteil an n,iso-Dialdehyd +
Anteil an iso,iso-Dialdehyd

Für den Zweck der Erläuterung der vorliegenden Erfindung umfasst der Ausdruck 'Alkyl' geradkettige und verzweigte Alkylgruppen. Vorzugsweise handelt es sich dabei um geradkettige oder verzweigte $C_1$-$C_{20}$-Alkyl, bevorzugterweise $C_1$-$C_{12}$-Alkyl-, besonders bevorzugt $C_1$-$C_8$-Alkyl- und ganz besonders bevorzugt $C_1$-$C_4$-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im allgemeinen 1, 2, 3, 4 oder 5, bevorzugt 1, 2 oder 3 und besonders bevorzugt 1 Substituenten, ausgewählt aus den Gruppen Cycloalkyl, Aryl, Hetaryl, Halogen, $NE^1E^2$, $NE^1E^2E^{3+}$, Carboxyl, Carboxylat, $-SO_3H$ und Sulfonat, tragen können.

Der Ausdruck "Alkylen" im Sinne der vorliegenden Erfindung steht für geradkettige oder verzweigte Alkandiyl-Gruppen mit 1 bis 4 Kohlenstoffatomen.

Der Ausdruck "Cycloalkyl" umfasst im Sinne der vorliegenden Erfindung unsubstituierte als auch substituierte Cycloalkylgruppen, vorzugsweise $C_5$- bis $C_7$-Cycloalkylgruppen, wie Cyclopentyl, Cyclohexyl oder Cycloheptyl, die im Falle einer Substitution, im allgemeinen 1, 2, 3, 4 oder 5, bevorzugt 1, 2 oder 3 und besonders bevorzugt 1 Substituenten, ausgewählt aus den Gruppen Alkyl, Alkoxy und Halogen, tragen können.

Der Ausdruck "Heterocycloalkyl" im Sinne der vorliegenden Erfindung umfasst gesättigte, cycloaliphatische Gruppen mit im allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, $COOR^f$, $COO^-M^+$ und $NE^1E^2$, bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetra-hydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl, wobei diese Arylgruppen im Falle einer Substitution im allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxyl, Carboxylat, Trifluormethyl, $-SO_3H$, Sulfonat, $NE^1E^2$, Alkylen-$NE^1E^2$, Nitro, Cyano oder Halogen, tragen können.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, sowie die Untergruppe der "Pyrrolgruppe", wobei diese heterocycloaromatischen Gruppen im Falle einer Substitution im allgemeinen 1, 2 oder 3 Substituenten, ausgewählt aus den Gruppen Alkyl, Alkoxy, Carboxyl, Carboxylat, $-SO_3H$, Sulfonat, $NE^1E^2$, Alkylen-$NE^1E^2$, Trifluormethyl oder Halogen, tragen können.

Der Ausdruck "Pyrrolgruppe" steht im Sinne der vorliegenden Erfindung für eine Reihe unsubstituierter oder substituierter, heterocycloaromatischer Gruppen, die strukturell vom Pyrrolgrundgerüst abgeleitet sind und ein pyrrolisches Stickstoffatom im Heterocyclus enthalten, das kovalent mit anderen Atomen, beispielsweise einem Pnicogenatom, verknüpft werden kann. Der Ausdruck "Pyrrolgruppe" umfasst somit die unsubstituierten oder substituierten Gruppen Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, die im Falle einer Substitution im allgemeinen 1, 2 oder 3, vorzugsweise 1 oder 2, besonders bevorzugt 1 Substituenten, ausgewählt aus den Gruppen Alkyl, Alkoxy, Acyl, Carboxyl, Carboxylat, $-SO_3H$, Sulfonat, $NE^1E^2$, Alkylen-$NE^1E^2$, Trifluormethyl oder Halogen, tragen können.

Dementsprechend umfasst der Ausdruck "Bispyrrolgruppe" im Sinne der vorliegenden Erfindung zweibindige Gruppen der Formel

Py-I-Py,

die zwei durch direkte chemische Bindung oder Alkylen-, Oxa-, Thio-, Imino-, Silyl oder Alkyliminogruppen vermittelte Verknüpfung, verbundene Pyrrolgruppen enthalten, wie die Bisindoldiyl-Gruppe der Formel

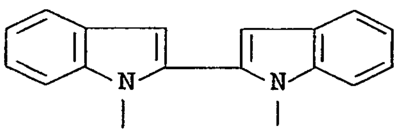

als Beispiel für eine Bispyrrolgruppe, die zwei direkt verknüpfte Pyrrolgruppen, in diesem Falle Indolyl, enthält, oder die Bispyrroldiyl-methan-Gruppe der Formel

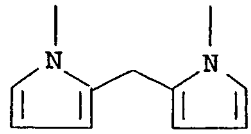

als Beispiel für eine Bispyrrolgruppe, die zwei über eine Methylengruppe verknüpfte Pyrrolgruppen, in diesem Falle Pyrrolyl, enthält. Wie die Pyrrolgruppen können auch die Bispyrrolgruppen unsubstituiert oder substituiert sein und im Falle einer Substitution pro Pyrrolgruppeneinheit im Allgemeinen 1, 2 oder 3, vorzugsweise 1 oder 2, insbesondere 1 Substituenten, ausgewählt aus Alkyl, Alkoxy, Carboxyl, Carboxylat, $-SO_3H$, Sulfonat, $NE^1E^2$, Alkylen-$NE^1E^2$, Trifluormethyl oder Halogen, tragen, wobei bei diesen Angaben zur Anzahl möglicher Substituenten die Verknüpfung der Pyrrolgruppeneinheiten durch direkte chemische Bindung oder durch die mittels der vorstehend genannten Gruppen vermittelte Verknüpfung nicht als Substitution betrachtet wird. Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit $C_1$-$C_4$-Alkanolen, wie Methanol, Ethanol, n-Propanol, Isopropanol, n-Butanol, sec.-Butanol und tert.-Butanol.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propionyl-, Butyryl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen $NE^1E^2$ und $NE^4E^5$ stehen vorzugsweise für N,N-Di-methylamino, N,N-Diethylamino, N,N-Dipropylamino, N,N-Diisopro-pylamino, N,N-Di-n-butylamino, N,N-Di-t.-butylamino, N,N-Dicyclo-hexylamino oder N,N-Diphenylamino.

Halogen steht für Fluor, Chlor, Brom und Iod, bevorzugt für Fluor, Chlor und Brom.

$M^+$ steht für ein Kationäquivalent, d. h. für ein einwertiges Kation oder den einer positiven Einfachladung entsprechenden Anteil eines mehrwertigen Kations. Das Kation $M^+$ dient lediglich als Gegenion zur Neutralisation negativ geladener Substituentengruppen, wie der COO- oder der Sulfonat-Gruppe und kann im Prinzip beliebig gewählt werden. Vorzugsweise werden deshalb Alkalimetall-, insbesondere $Na^+$-, $K^+$-, $Li^+$-Ionen oder Onium-Ionen, wie Ammonium-, Mono-, Di-, Tri-, Tetraalkylammonium-, Phosphonium-, Tetraalkylphosphonium oder Tetraarylphosphonium-Ionen verwendet.

Entsprechendes gilt für das Anionäquivalent $X^-$, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions, wobei im Allgemeinen Halogenid-Ionen X- bevorzugt sind, insbesondere Chlorid und Bromid.

Die Werte für x stehen für eine ganze Zahl von 1 bis 240, vorzugsweise für eine ganze Zahl von 3 bis 120.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme.

Y stellt eine chemische Bindung, also den Anknüpfungspunkt der Brückengruppe Q an die Gruppen -O-, oder im Falle wenn a und/oder b gleich 0 ist, an die Gruppen $PnR^1R^2$ bzw. $PnR^3R^4$ dar.

In der Brückengruppe Q können die Gruppen $A^1$ und $A^2$ im Allgemeinen unabhängig voneinander für O, S, $SiR^aR^b$, $NR^c$ oder $CR^dR^e$ stehen, wobei die Substituenten $R^a$, $R^b$ und $R^c$ im Allgemeinen unabhängig voneinander die Bedeutung Wasserstoff, Alkyl, Cycloalkyl, Heterocycloalkyl, Aryl oder Hetaryl haben können, wohingegen die Gruppen $R^d$ und $R^e$ unabhängig voneinander für Wasserstoff, Alkyl, Cycloalkyl, Heterocycloalkyl, Aryl oder Hetaryl stehen oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden können.

D ist eine zweibindige Brückengruppe, die im Allgemeinen ausgewählt ist aus den Gruppen

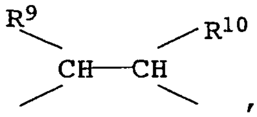,
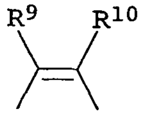,
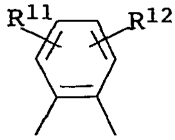,
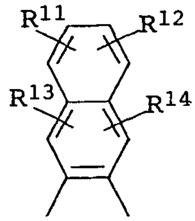

in denen $R^9$ und $R^{10}$ unabhängig voneinander für Wasserstoff, Alkyl, Cycloalkyl, Aryl, Halogen, Trifluormethyl, Carboxyl, Carboxylat oder Cyano stehen oder miteinander zu einer $C_3$-$C_4$-Alkylengruppe verbunden sind und $R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carboxylat, Cyano, Alkoxy, $SO_3H$, Sulfonat, $NE^1E^2$, Alkylen-$NE^1E^2E^{3+}X^-$, Aryl oder Nitro stehen können. Vorzugsweise stehen die Gruppen $R^9$ und $R^{10}$ für Wasserstoff, $C_1$-$C_{10}$-Alkyl oder

Carboxylat und die Gruppen $R^{11}$, $R^{12}$, $R^{13}$ und $R^{14}$ für Wasserstoff, $C_1$-$C_{10}$-Alkyl, Halogen, insbesondere Fluor, Chlor oder Brom, Trifluormethyl, $C_1$-$C_4$-Alkoxy, Carboxylat, Sulfonat oder $C_6$-$C_{14}$-Aryl. Besonders bevorzugt stehen $R^9$, $R^{10}$, $R^{11}$, $R^{12}$, $R^{13}$ und $R^{14}$ für Wasserstoff. Für den Einsatz in einem wässrigen Reaktionsmedium sind solche Pnicogenchelatverbindungen bevorzugt, in denen 1, 2 oder 3, vorzugsweise 1 oder 2, insbesondere 1 der Gruppen $R^{11}$, $R^{12}$, $R^{13}$ und/oder $R^{14}$ für eine $COO^-Me^+$, eine $SO_3^-M^+$ oder eine $NE^1E^2E^{3+}X^-$-Gruppe stehen, wobei $M^+$ und X- die vorstehend genannte Bedeutung haben.

Besonders bevorzugte Brückengruppen D sind die Ethylengruppe

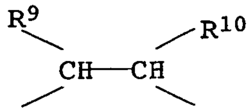

und die 1,2-Phenylengruppe

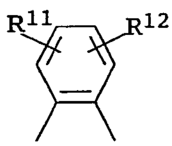

Wenn $R^d$ mit einer weiteren Gruppe $R^d$ oder $R^e$ mit einer weiteren Gruppe $R^e$ eine intramolekulare Brückengruppe D bildet, dann ist der Index c in diesem Falle gleich 1.

Bevorzugte Brückengruppen Q sind außer denen mit Triptycen-artigem Kohlenstoffgerüst solche, in denen der Index c für 0 steht und die Gruppen $A^1$ und $A^2$ ausgewählt sind aus den Gruppen O, S und $CR^dR^e$, insbesondere unter O, S, der Methylengruppe ($R^d = R^e = H$), der Dimethylmethylengruppe ($R^d = R^e = CH_3$), Diethylmethylengruppe ($R^d = R^e = C_2H_5$), der Di-n-propyl-methylengruppe ($R^d = R^e$ = n-Propyl) oder der Di-n-butylmethylengruppe ($R^d = R^e$ = n-Butyl). Insbesondere sind solche Brückengruppen Q bevorzugt, in denen $A^1$ von $A^2$ verschieden ist, wobei $A^1$ bevorzugt eine $CR^dR^e$-Gruppe und $A^2$ bevorzugt eine O- oder S-Gruppe, besonders bevorzugt eine Oxagruppe O ist.

Besonders bevorzugte Brückengruppen Q sind somit solche, die aus einem Triptycen-artigen oder Xanthen-artigen ($A^1$: $CR^dR^e$, $A^2$: O) Gerüst aufgebaut sind.

Die Substituenten $R^I$ $R^{II}$, $R^{III}$, $R^{IV}$, $R^V$ und $R^{VI}$ sind vorzugsweise ausgewählt unter Wasserstoff, Alkyl, Alkoxy, Cycloalkyl, Heterocycloalkyl, Aryl und Hetaryl. Nach einer ersten bevorzugten Ausführungsform stehen $R^I$, $R^{II}$, $R^{III}$, $R^{IV}$, $R^V$ und $R^{VI}$ für Wasserstofff. Nach einer weiteren bevorzugten Ausführungsform stehen $R^I$ und $R^{VI}$ unabhängig voneinander für $C_1$-$C_4$-Alkyl oder $C_1$-$C_4$-Alkoxy. Vorzugsweise sind $R^I$ und $R^{VI}$ ausgewählt unter Methyl, Ethyl, Isopropyl, tert-Butyl und Methoxy. Bevorzugt stehen in diesen Verbindungen $R^{II}$, $R^{III}$, $R^{IV}$ und $R^V$ für Wasserstoff. Nach einer weiteren bevorzugten Ausführungsform stehen $R^{II}$ und $R^V$ unabhängig voneinader für $C_1$-$C_4$-Alkyl oder $C_1$-$C_4$-Alkoxy. Vorzugsweise sind $R^{II}$ und $R^V$ ausgewählt unter Methyl, Ethyl, Isopropyl, tert-Butyl und Methoxy. Bevorzugt stehen in diesen Verbindungen $R^I$, $R^{III}$, $R^{IV}$ und $R^{VI}$ für Wasserstoff.

Wenn zwei benachbarte Reste, ausgewählt unter $R^I$, $R^{II}$, $R^{III}$, $R^{IV}$, $R^V$ und $R^{VI}$ für ein ankondensiertes, also anelliertes, Ringsystem stehen, so handelt es sich bevorzugt um Benzol- oder Naphthalinringe. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, $SO_3H$, Sulfonat, $NE^1E^2$, Alkylen-$NE^1E^2$, Trifluormethyl, Nitro, $COOR^f$, Alkoxycarbonyl, Acyl und Cyano. Anellierte Naphthalinringe sind vorzugsweise unsubstituiert oder weisen im nicht anellierten Ring und/oder im anellierten Ring insgesamt 1, 2 oder 3, insbesondere 1 oder 2 der zuvor bei den anellierten Benzolringen genannten Substituenten auf.

Ist der Einsatz der erfindungsgemäß eingesetzten Pnicogenchelat-verbindungen in einem wässrigen Hydroformylierungsmedium vorgesehen, steht wenigstens einer der Reste $R^I$, $R^{II}$, $R^{III}$, $R^{IV}$, $R^V$ und/oder $R^{VI}$ für eine polare (hydrophile) Gruppe, wobei dann in der Regel bei der Komplexbildung mit einem Gruppe VIII Metall wasserlösliche Pnicogenchelatkomplexe resultieren. Bevorzugt sind die polaren Gruppen ausgewählt unter $COOR^f$, $COO^-M^+$, $SO_3R^f$, $SO_3^-M^+$, $NE^1E^2$, Alkylen-$NE^1E^2$, $NE^1E^2E^{3+}X^-$, Alkylen-$NE^1E^2E^{3+}X^-$, $OR^f$, $SR^f$, $(CHR^gCH_2O)_xR^f$ oder $(CH_2CH_2N(E^1))_xR^f$, worin $R^f$ , $E^1$, $E^2$, $E^3$, $R^g$, $M^+$, $X^-$ und x die zuvor angegebenen Bedeutungen besitzen.

Die Brückengruppe Q ist über die chemische Bindung Y entweder direkt oder über eine Oxagruppe O mit den Gruppen $PnR^1R^2$ bzw. $PnR^3R^4$ verbunden.

Pn steht für ein Atom aus der Pnicogengruppe, ausgewählt aus Phosphor, Arsen oder Antimon. Besonders bevorzugt steht Pn für Phosphor.

Die einzelnen Pnicogenatome Pn der erfindungsgemäß eingesetzten Pnicogenchelatverbindungen sind jeweils über zwei kovalente Bindungen mit zwei Substituenten $R^1$ und $R^2$ bzw. $R^3$ und $R^4$ verbunden, wobei die Substituenten $R^1$, $R^2$, $R^3$ und $R^4$ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine $NE^1E^2$-Gruppe stehen können, mit der Maßgabe, dass $R^1$ und $R^3$ über das pyrrolische Stickstoffatom an das Pnicogenatom Pn gebundene Pyrrolgruppen sind. Vorteilhaft stehen auch die Substituenten $R^2$ und/oder $R^4$ für über das pyrrolische Stickstoffatom an das Pnicogenatom Pn gebundene Pyrrolgruppen. Weiterhin vorteilhaft kann der Substituent $R^1$ gemeinsam mit dem Substituenten $R^2$ und/oder der Substituent $R^3$ gemeinsam mit dem Substituenten $R^4$ eine über die pyrrolischen Stickstoffatome an das Pnicogenatom Pn gebundene Bispyrrolgruppe bilden.

Die Bedeutung der einzelnen im vorstehenden Absatz genannten Ausdrücke entspricht der eingangs gegebenen Definition.

In einer bevorzugten Ausführungsform wird in dem erfindungsgemäßen Verfahren ein Hydroformylierungskatalysator eingesetzt, in dem die Reste $R^1$, $R^2$, $R^3$ und $R^4$ unabhängig voneinander ausgewählt sind unter Gruppen der Formeln I.a bis I.k:

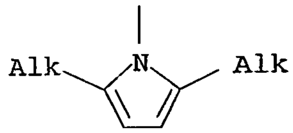

(I.a)

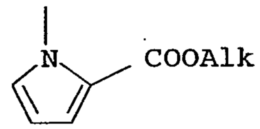

(I.b)

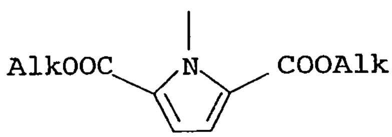

(I.c)

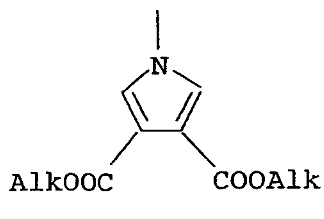

(I.d)

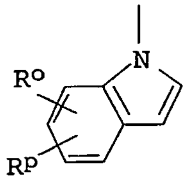

(I.e)

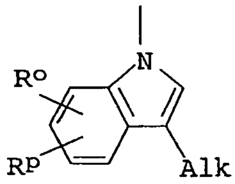

(I.f)

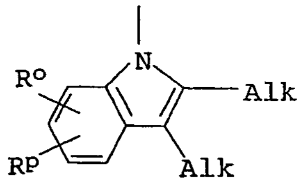

(I.g)

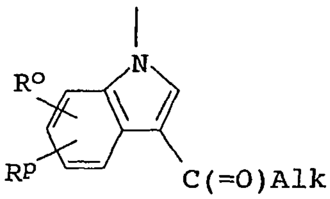

(I.h)

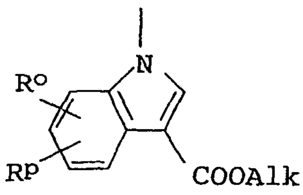

(I.i)

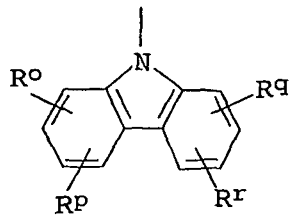

(I.k)

worin

Alk eine $C_1$-$C_4$-Alkylgruppe ist und

$R^o$, $R^p$, $R^q$ und $R^r$ unabhängig voneinander für Wasserstoff, $C_1$-$C_4$-Alkyl, $C_1$-$C_4$-Alkoxy, Acyl, Halogen, Trifluormethyl, $C_1$-$C_4$-Alkoxycarbonyl oder Carboxyl stehen.

Zur Veranschaulichung werden im Folgenden einige vorteilhafte Pyrrolgruppen aufgelistet:

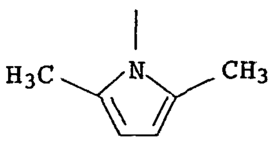

(I.a1)

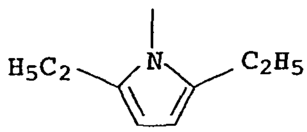

(I.a2)

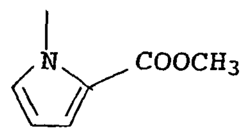

(I.b1)

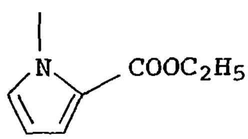

(I.b2)

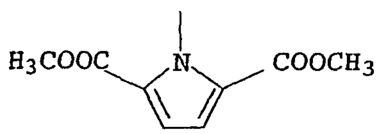

(I.c1)

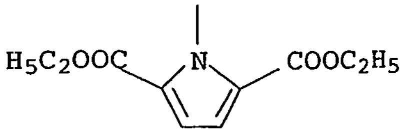

(I.c2)

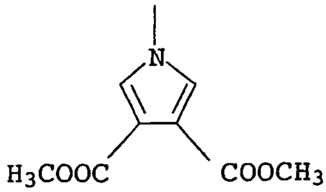

(I.d1)

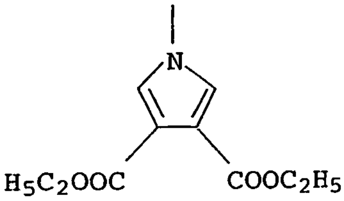

(I.d2)

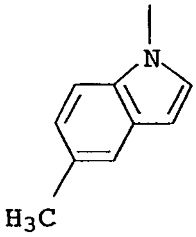

(I.e1)

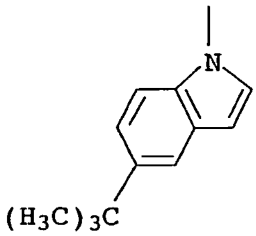

(I.e2)

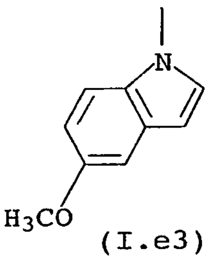

(I.e3)

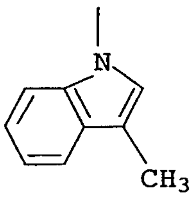

(I.f1)

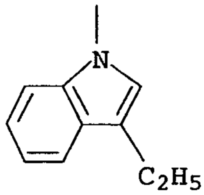

(I.f2)

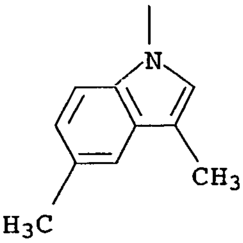

(I.f3)

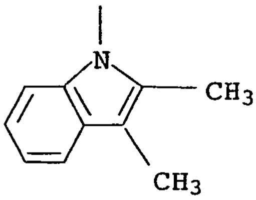

(I.g1)

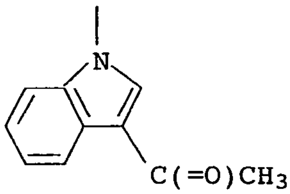

(I.h1)

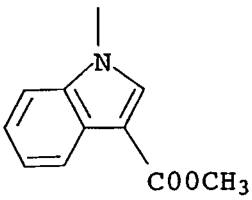

(I.i1)

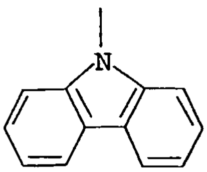

(I.k1)

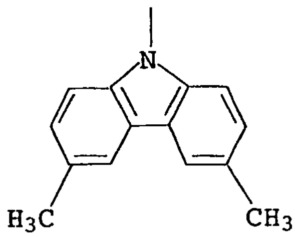

(I.k2)

Besonders vorteilhaft ist die 3-Methylindolylgruppe (Skatolylgruppe) der Formel I.f1. Hydroformylierungskatalysatoren auf Basis von Liganden, die eine oder mehrere 3-Methylindolylgruppe(n) an das Phosphoratom gebunden aufweisen, zeichnen sich durch eine besonders hohe Stabilität und somit besonders lange Katalysatorstandzeiten aus.

In einer weiteren vorteilhaften Ausgestaltung der vorliegenden Erfindung kann der Substituent $R^1$ gemeinsam mit dem Substituenten $R^2$ oder der Substituent $R^3$ gemeinsam mit dem Substituenten $R^4$ eine über das pyrrolische Stickstoffatom an das Pnicogenatom Pn gebundene Pyrrolgruppe enthaltende zweibindige Gruppe der Formel

Py-I-W

bilden,
worin

| | |
|---|---|
| Py | eine Pyrrolgruppe ist, |
| I | für eine chemische Bindung oder für O, S, $SiR^aR^b$, $NR^c$ oder $CR^hR^i$ steht, |
| W und | für Cycloalkyl, Cycloalkoxy, Aryl, Aryloxy, Hetaryl oder Hetaryloxy steht |
| $R^h$ und $R^i$ | unabhängig voneinander für Wasserstoff, Alkyl, Cyclo- alkyl, Heterocycloalkyl, Aryl oder Hetaryl stehen, |

wobei die hierbei verwendeten Bezeichnungen die eingangs erläuterte Bedeutung haben.

Bevorzugte zweibindige Gruppen der Formel

Py-I-W

sind z. B.

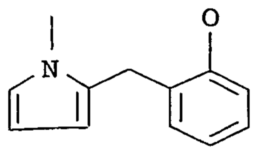 ; 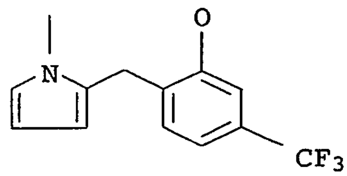 ; 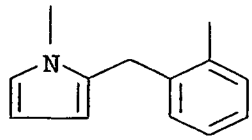 ;

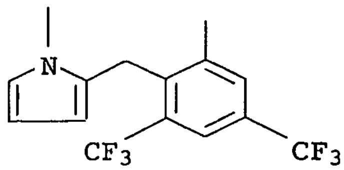 ; 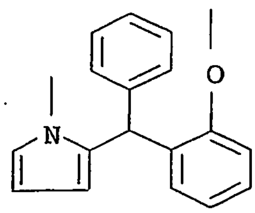 ; 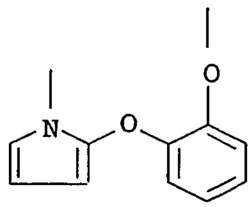 ;

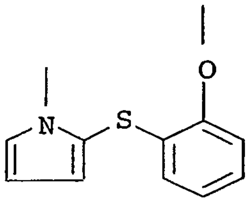 ; 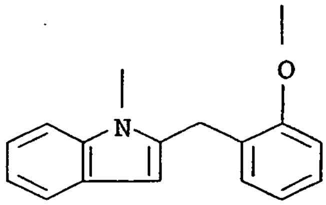

Bevorzugt werden in dem erfindungsgemäßen Verfahren Hydroformylierungskatalysatoren eingesetzt, die wenigstens einen Liganden der Formel I umfassen, worin der Substituent $R^1$ gemeinsam mit dem Substituenten $R^2$ oder der Substituent $R^3$ gemeinsam mit dem Substituenten $R^4$ eine Bispyrolgruppe der Formel

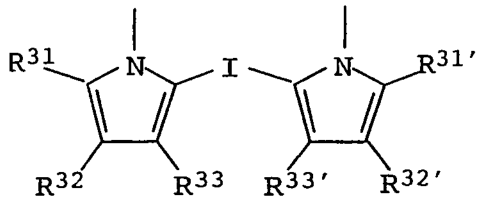

oder

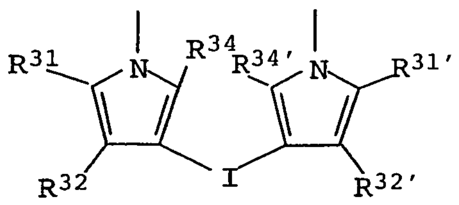

bildet, worin

I für eine chemische Bindung oder für O, S, $SiR^aR^b$, $NR^c$ oder ge- gebenenfalls substituiertes $C_1$-$C_{10}$-Alkylen, bevorzugt $CR^hR^i$, steht, worin $R^a$, $R^b$, $R^c$, $R^h$ und $R^i$ unabhängig voneinander für Wasserstoff, Alkyl, Cycloalkyl, Heterocycloalkyl, Aryl oder Hetaryl stehen,

$R^{31}$, $R^{31'}$, $R^{32}$, $R^{32'}$, $R^{33}$, $R^{33'}$, $R^{34}$ und $R^{34'}$ unabhängig voneinan- der für Wasserstoff, Alkyl, Cycloalkyl, Heterocycloalkyl, Aryl, Hetaryl, $W'COOR^a$, $W'COO^-M^+$, $W'(SO_3)R^f$, $W'(SO_3)^-M^+$, $W'PO_3(R^f)(R^g)$, $W'(PO_3)^{2-}(M^+)_2$, $W'NE^1E^2$, $W'(NE^1E^2E^3)^+X^-$, $W'OR^f$, $W'SR^f$, $(CHR^gCH_2O)_xR^f$, $(CH_2NE^1)_xR^f$, $(CH_2CH_2NE^1)_xR^f$, Halogen, Trifluormethyl, Nitro, Acyl oder Cyano stehen, worin

W' für eine Einfachbindung, ein Heteroatom oder eine zweiwertige verbrückende Gruppe mit 1 bis 20 Brük- kenatomen steht,

$R^f$, $E^1$, $E^2$, $E^3$ jeweils gleiche oder verschiedene Reste, ausgewählt unter Wasserstoff, Alkyl, Cycloalkyl oder Aryl bedeuten,

$R^g$ für Wasserstoff, Methyl oder Ethyl steht,

$M^+$ für ein Kationäquivalent steht,

$X^-$ für ein Anionäquivalent steht und

x für eine ganze Zahl von 1 bis 240 steht,

wobei jeweils zwei benachbarte Reste $R^{31}$ und $R^{32}$ und/oder $R^{31'}$ und $R^{32'}$ zusammen mit den Kohlenstoffatomen des Pyrrolrings, an die sie gebunden sind, auch für ein kondensiertes Ringsystem mit 1, 2 oder 3 weiteren Ringen stehen können.

Vorzugsweise steht I für eine chemische Bindung oder eine $C_1$-$C_4$-Alkylengruppe, besonders bevorzugt eine Methylengruppe.

Zur Veranschaulichung werden im Folgenden einige vorteilhafte "Bispyrrolylgruppen" aufgelistet:

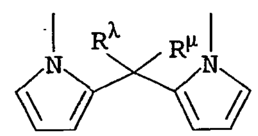

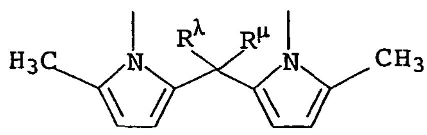

a: $R^\lambda$, $R^\mu$ = H

b: $R^\lambda$ = H

$R^\mu$ = $C_6H_5$

c: $(R^\lambda + R^\mu)$ = $C_4H_8$

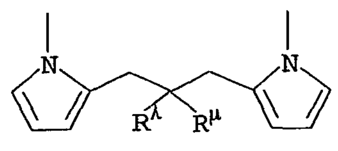

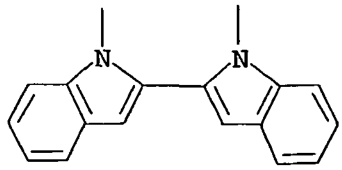

In einer bevorzugten Ausführungsform sind die erfindungsgemäß eingesetzten Pnicogenchelatverbindungen ausgewählt unter Verbindungen der allgemeinen Formel II

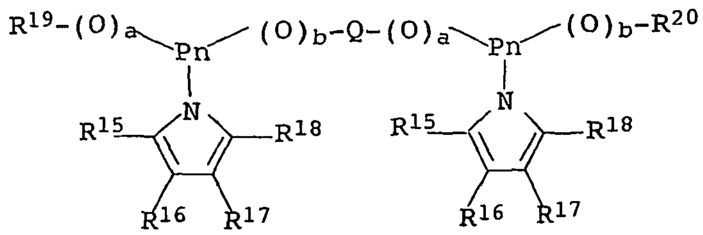

(II)

worin

R^15, R^16, R^17 und R^18 unabhängig voneinander für Wasserstoff, Al- kyl, Cycloalkyl, Heterocycloalkyl, Aryl, Hetaryl, $W'COOR^k$, $W'COO^-M^+$, $W'(SO_3)R^k$, $W'(SO_3)^-M^+$, $W'PO_3(R^k)(R^1)$, $W'(PO_3)^{2-}(M^+)_2$, $W'NE^4E^5$, $W'(NE^4E^5E^6)^+X^-$, $W'OR^k$, $W'SR^K$, $(CHR^1CH_2O)_yR^k$, $(CH_2NE^4)_yR^k$, $(CH_2CH_2NE^4)_yR^k$, Halogen, Trifluormethyl, Nitro, Acyl oder Cyano stehen, worin

W' für eine Einfachbindung, ein Heteroatom oder eine zwei- wertige verbrückende Gruppe mit 1 bis 20 Brückenatomen steht,

$R^k$, $E^4$, $E^5$, $E^6$ jeweils gleiche oder verschiedene Reste, ausge- wählt unter Wasserstoff, Alkyl, Cycloalkyl oder Aryl be- deuten,

$R^1$ für Wasserstoff, Methyl oder Ethyl steht,

$M^+$ für ein Kationäquivalent steht,

$X^-$ für ein Anionäquivalent steht und

y für eine ganze Zahl von 1 bis 240 steht,

wobei jeweils zwei benachbarte Reste $R^{15}$, $R^{16}$, $R^{17}$ und $R^{18}$ zusammen mit den Kohlenstoffatomen des Pyrrolrings, an die sie gebunden sind, auch für ein kondensiertes Ringsystem mit 1, 2 oder 3 weiteren Ringen stehen können, mit der Maßgabe, dass wenigstens einer der Reste $R^{15}$, $R^{16}$, $R^{17}$ oder $R^{18}$ nicht für Wasserstoff steht, und dass $R^{19}$ und $R^{20}$ nicht mit einander verknüpft sind,

$R^{19}$ und $R^{20}$ unabhängig voneinander für Cycloalkyl, Heterocycloal- kyl, Aryl oder Hetaryl stehen,

a und b unabhängig voneinander die Zahl 0 oder 1 bedeuten,

Pn für ein Pnicogenatom, ausgewählt aus den Elementen Phosphor, Arsen oder Antimon, bevorzugt für Phosphor, steht,

Q eine Brückengruppe wie zuvor definiert ist.

Bevorzugt stehen in den Verbindungen der Formel II die Pnicogenatome Pn beide für Phosphor.

Bezüglich geeigneter und bevorzugter Ausführungsformen der Brückengruppe Q wird auf die vorherigen Ausführungen in vollem Umfang Bezug genommen.

Die Reste $R^{15}$ bis $R^{18}$ können jeweils unabhängig voneinander gleiche oder verschiedene Bedeutungen aufweisen.

Bevorzugt sind Verbindungen der allgemeinen Formel II, wobei in den Pyrrolgruppen jeweils einer oder zwei der Reste $R^{15}$, $R^{16}$, $R^{17}$ und $R^{18}$ für einen der zuvor genannten, von Wasserstoff verschiedenen Substituenten stehen und die übrigen für Wasserstoff stehen. Bevorzugt sind Verbindungen der Formel II, bei denen die Pyrrolgruppen in 2-Position, 2,5-Position oder 3,4-Position einen von Wasserstoff verschiedenen Substituenten tragen.

Vorzugsweise sind die von Wasserstoff verschiedenen Substituenten $R^{15}$ bis $R^{18}$ unabhängig voneinander ausgewählt unter $C_1$- bis $C_8$-, vorzugsweise $C_1$- bis $C_4$-Alkyl, speziell Methyl, Ethyl, Isopropyl und tert.-Butyl, Alkoxycarbonyl, wie Methoxycarbonyl, Ethoxycarbonyl, Isopropyloxycarbonyl und tert.-Butyloxycarbonyl sowie Trifluormethyl.

Bevorzugt sind Verbindungen der allgemeinen Formel II, worin die Reste $R^{15}$ und $R^{16}$ und/oder $R^{17}$ und $R^{18}$ zusammen mit den Kohlenstoffatomen des Pyrrolrings, an die sie gebunden sind, für ein kondensiertes Ringsystem mit 1, 2 oder 3 weiteren Ringen stehen. Wenn $R^{15}$ und $R^{16}$ und/oder $R^{17}$ und $R^{18}$ für ein ankondensiertes, also anelliertes Ringsystem stehen, so handelt es sich bevorzugt um Benzol- oder Naphthalinringe. Anellierte Benzolringe sind vorzugsweise unsubstituiert und weisen 1, 2 oder 3, insbesondere 1 oder 2 Substituenten auf, die ausgewählt sind unter Alkyl, Alkoxy, Halogen, $SO_3H$, Sulfonat, $NE^4E^5$, Alkylen-$NE^4E^5$, Trifluormethyl, Nitro, $COOR^k$, Alkoxycarbonyl, Acyl und Cyano. Anellierte Naphthalinringe sind vorzugsweise unsubstituiert oder weisen im nichtanellierten Ring und/oder im anellierten Ring je 1, 2 oder 3, insbesondere 1 oder 2 der zuvor bei den anellierten Benzolringen genannten Substituenten auf. Wenn $R^{15}$ und $R^{16}$ für ein ankondensiertes Ringsystem stehen, so stehen $R^{17}$ und $R^{18}$ vorzugsweise für Wasserstoff oder steht $R^{18}$ für Wasserstoff und $R^{17}$ für einen Substituenten, der ausgewählt ist unter $C_1$- bis $C_8$-Alkyl, vorzugsweise $C_1$- bis $C_4$-Alkyl, speziell Methyl, Ethyl, Isopropyl oder tert.-Butyl.

Ist der Einsatz der Verbindungen der Formel II in einem wässrigen Hydroformylierungsmedium vorgesehen, steht wenigstens einer der Reste $R^{15}$, $R^{16}$, $R^{17}$ und/oder $R^{18}$ für eine polare (hydrophile) Gruppe, wobei dann in der Regel bei der Komplexbildung mit einem Gruppe VIII Metall wasserlösliche Komplexe resultieren. Bevorzugt sind die polaren Gruppen ausgewählt unter $COOR^k$, $COO^-M^+$, $SO_3R^k$, $SO_3^-M^+$, $NE^4E^5$, Alkylen-$NE^4E^5$, $NE^4E^5E^{6+}X^-$, Alkylen-$NE^4E^5E^{6+}X^-$, $OR^k$, $SR^k$, $(CHR^1CH_2O)_yR^k$ oder $(CH_2CH_2N(E^4))_yR^k$, worin $R^k$, $E^4$, $E^5$, $E^6$, $R^1$, $M^+$, $X^-$ und y die zuvor angegebenen Bedeutungen besitzen.

Vorzugsweise sind die Verbindungen der Formel II ausgewählt unter Verbindungen der allgemeinen Formeln II.1 bis II.3

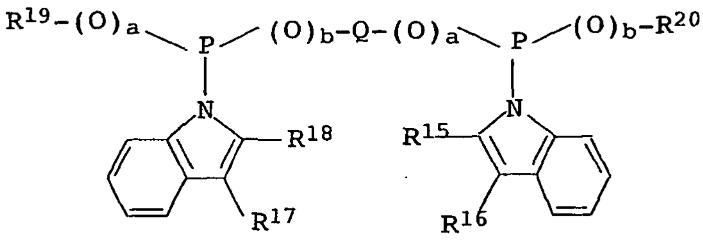

(II.1)

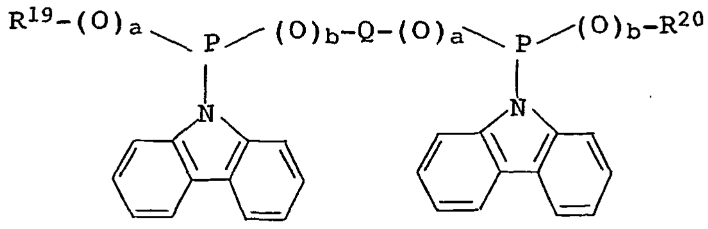

(II.2)

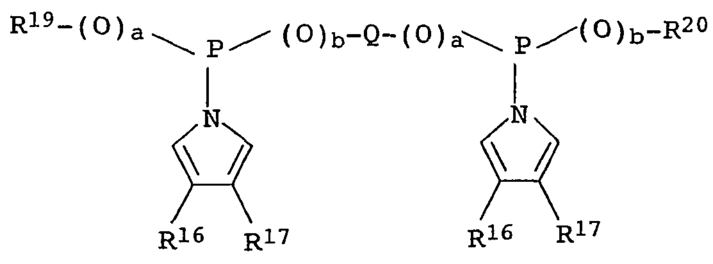

(II.3)

worin

| | |
|---|---|
| $R^{15}$, $R^{16}$, $R^{17}$, $R^{18}$, Q, a und b | die zuvor angegebenen Bedeutungen besitzen, wobei in der Formel II.3 wenigstens einer der Reste $R^{16}$ oder $R^{17}$ nicht für Wasserstoff steht, |
| $R^{19}$ und $R^{20}$ | unabhängig voneinander für Cycloalkyl, Heterocycloal- kyl, Aryl oder Hetaryl stehen. |

Vorzugsweise stehen in den Verbindungen der Formel II.1 die Reste $R^{15}$ bis $R^{18}$ alle für Wasserstoff. Des Weiteren vorzugsweise stehen $R^{15}$ und $R^{18}$ für Wasserstoff und sind $R^{16}$ und $R^{17}$ ausgewählt unter $C_1$-$C_8$-Alkyl, vorzugsweise $C_1$-$C_4$-Alkyl, wie Methyl, Ethyl, Isopropyl und tert.-Butyl.

Vorzugsweise sind in den Verbindungen der Formel II.3 die Reste $R^{16}$ und $R^{17}$ ausgewählt unter $C_1$-$C_8$-Alkyl, besonders bevorzugt $C_1$-$C_4$-Alkyl, wie Methyl, Ethyl, Isopropyl und tert.-Butyl, sowie $COOR^k$, worin $R^k$ für $C_1$-$C_4$-Alkyl, wie Methyl, Ethyl, Isopropyl und tert.-Butyl steht.

Lediglich zur Veranschaulichung der erfindungsgemäß eingesetzten Pnicogenchelatverbindung werden im folgenden einige vorteilhafte Verbindungen aufgelistet:

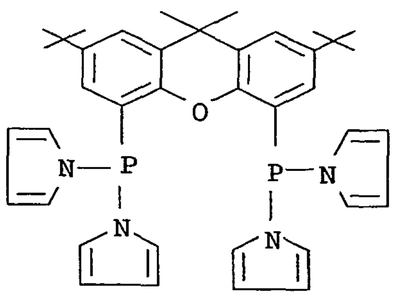

1

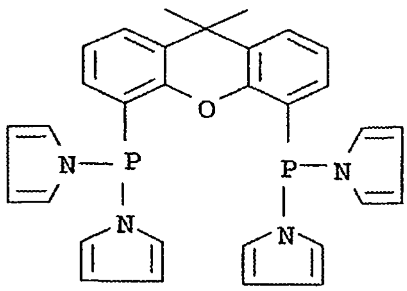

2

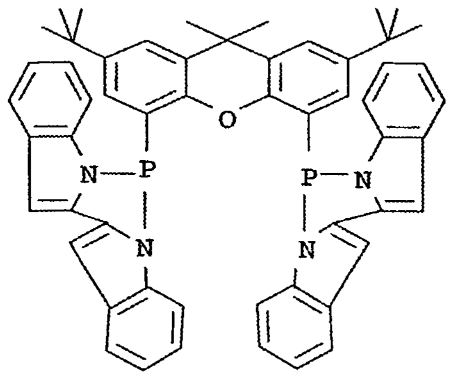

3

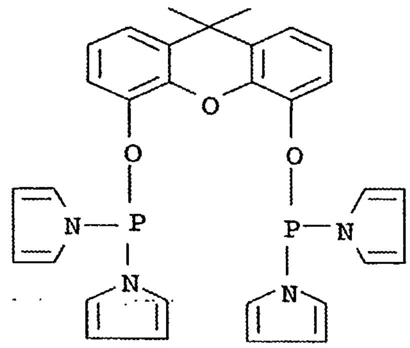

4

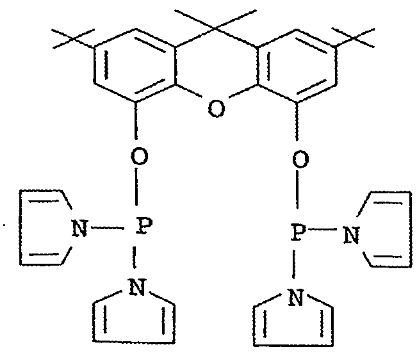

5

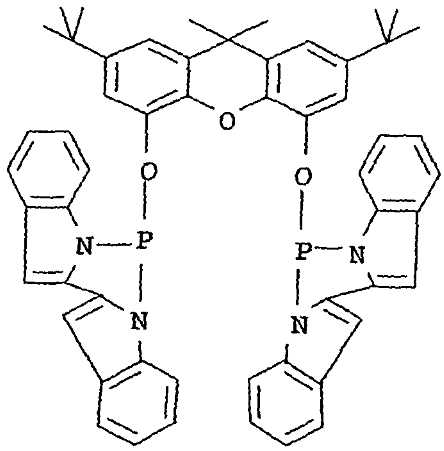

6

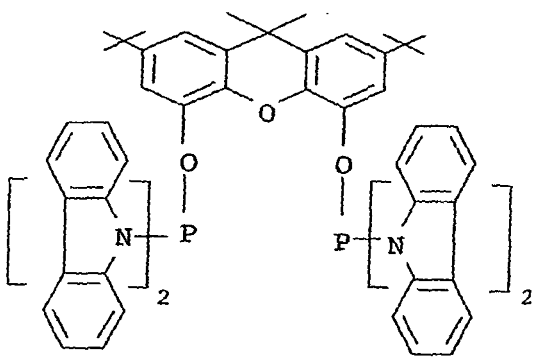

7

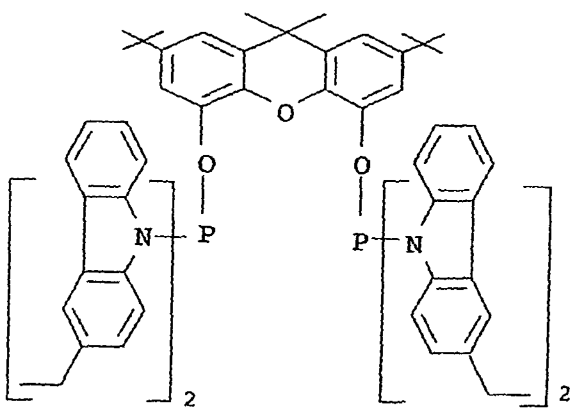

8

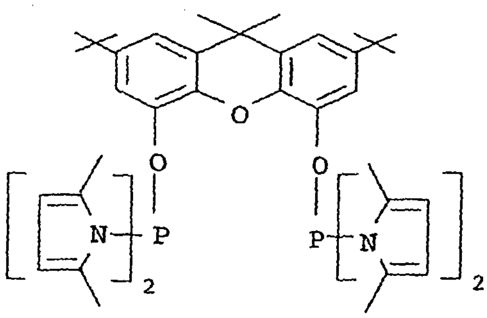

9

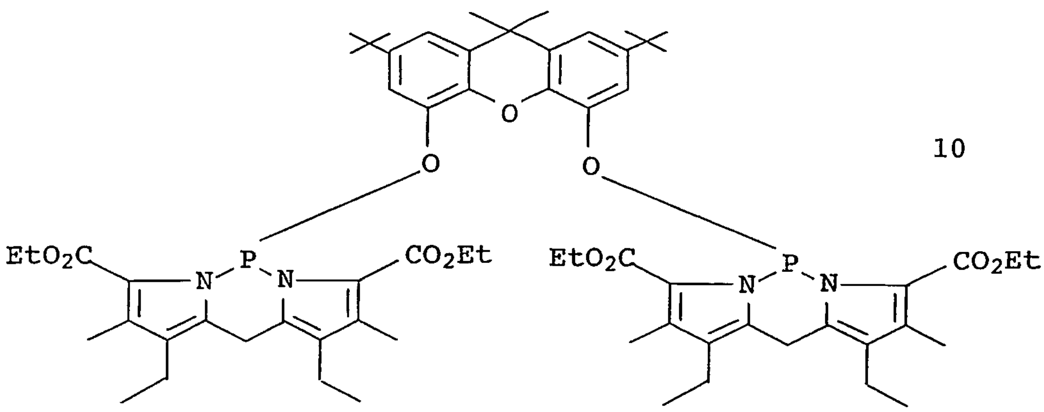

10

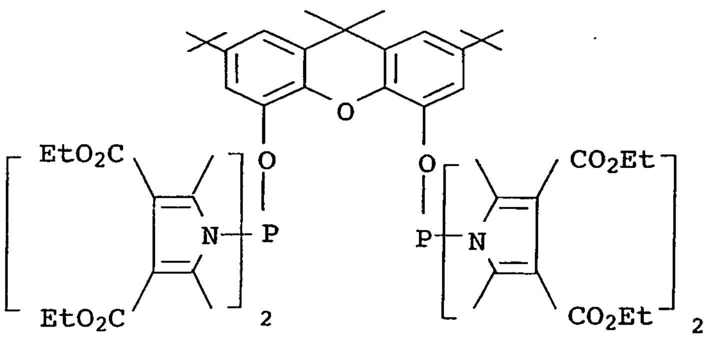

11

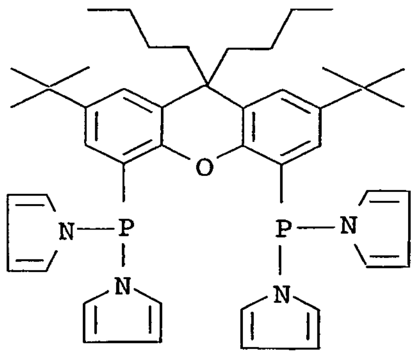

12

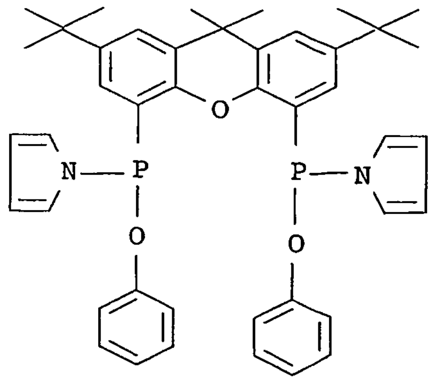

13

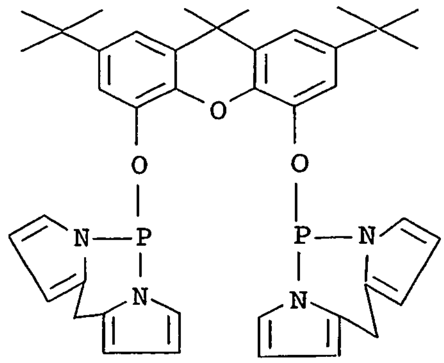

14

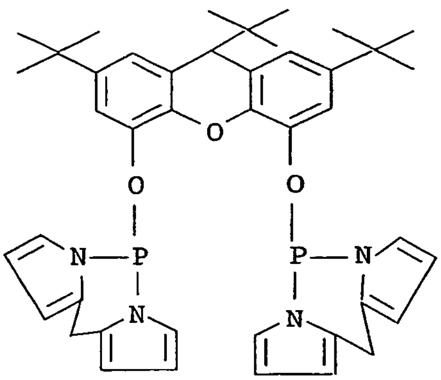

15

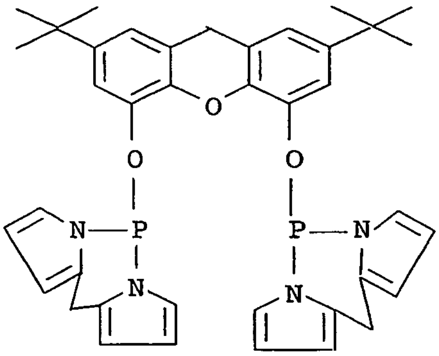

16

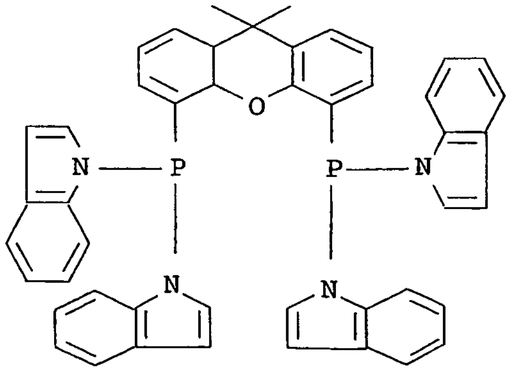

17

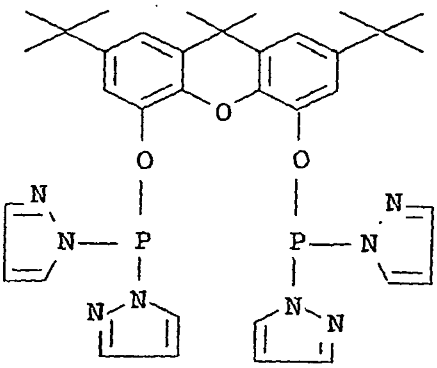

18

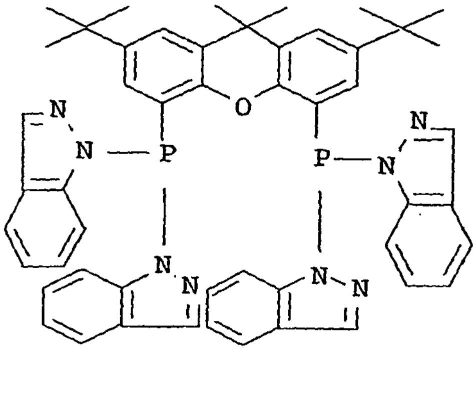

19

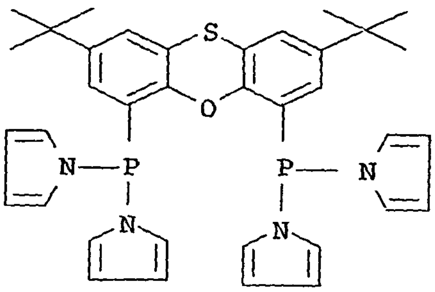

20

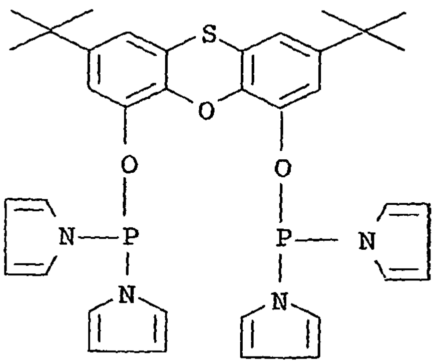

21

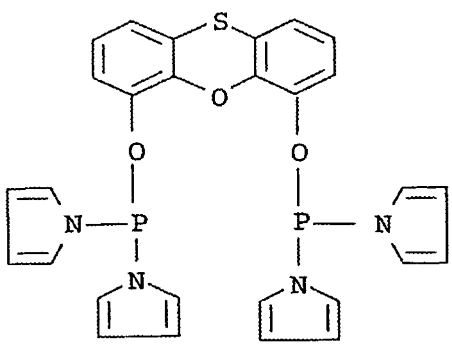

22

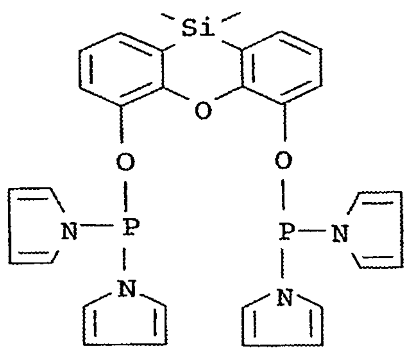

23

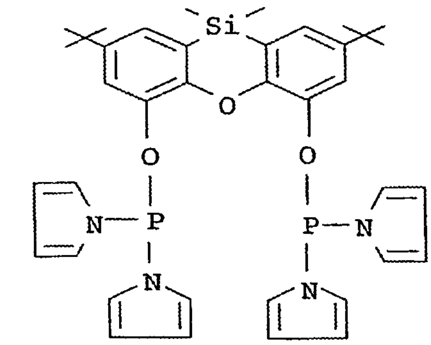

24

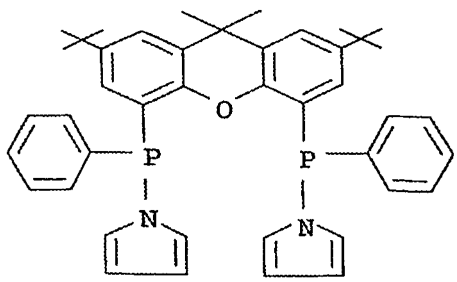

25

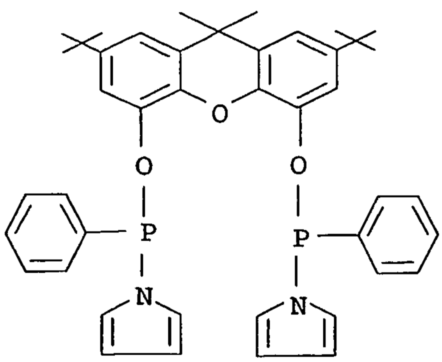

26

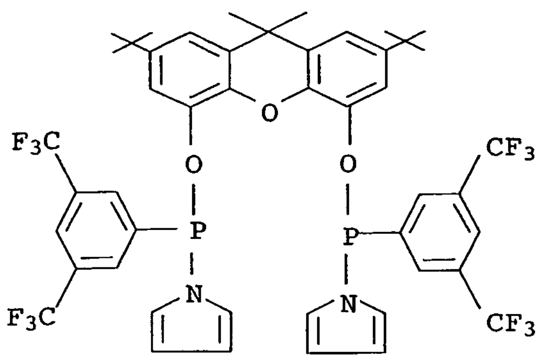

27

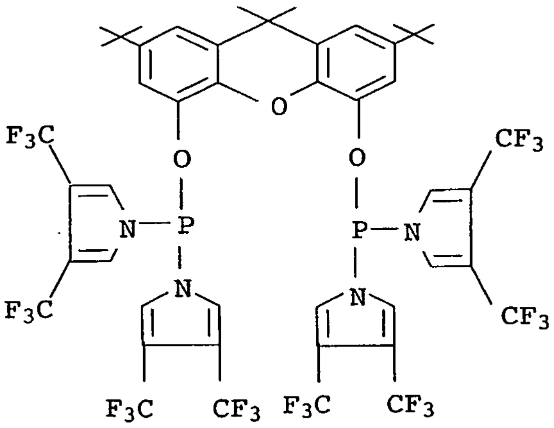

28

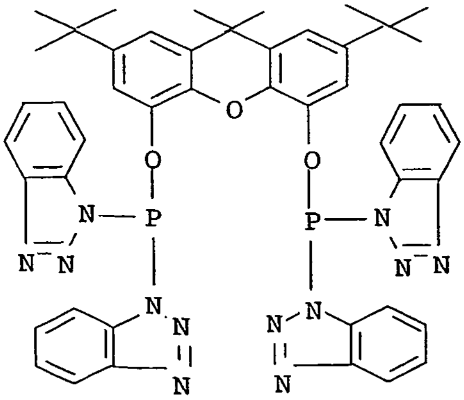

29

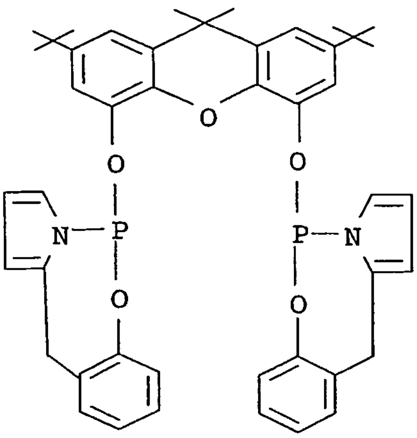

30

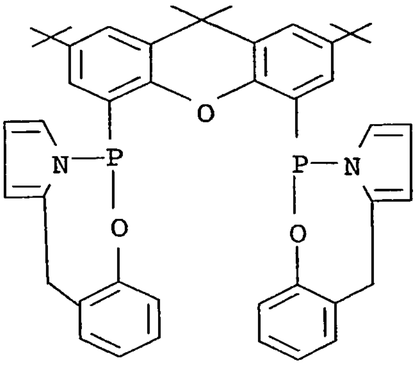

31

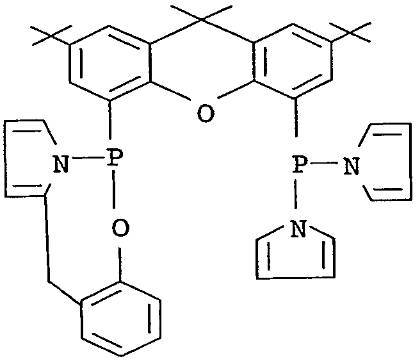

32

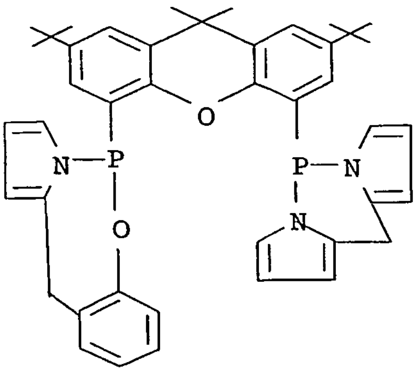

33

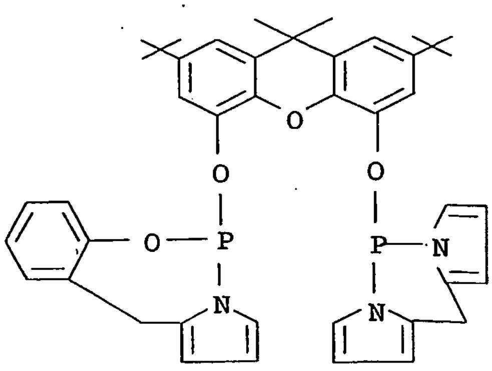

34

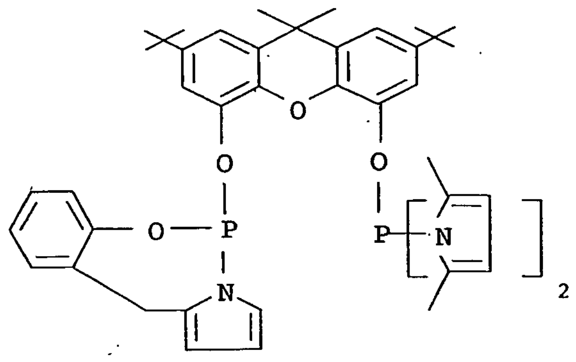

35

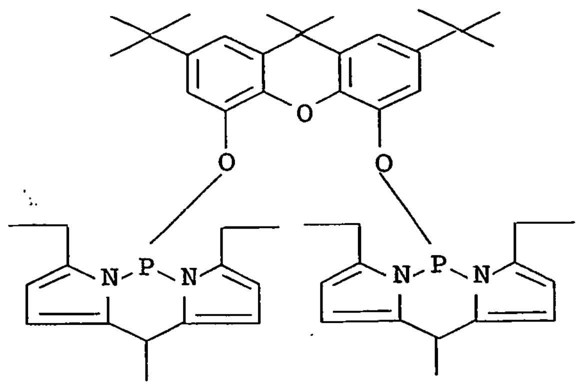

36

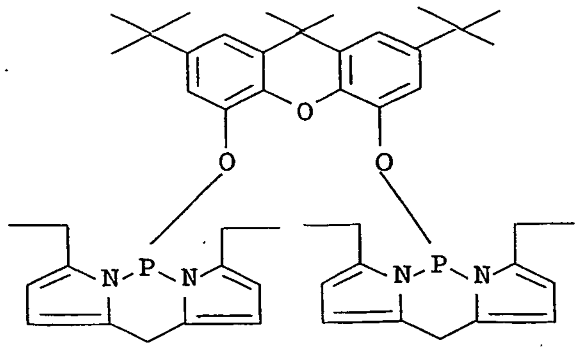

37

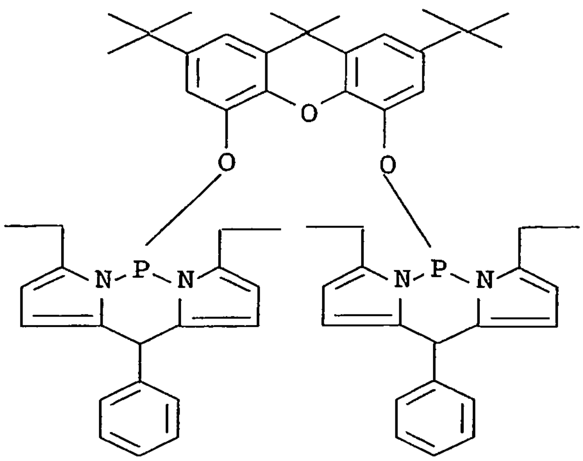

38

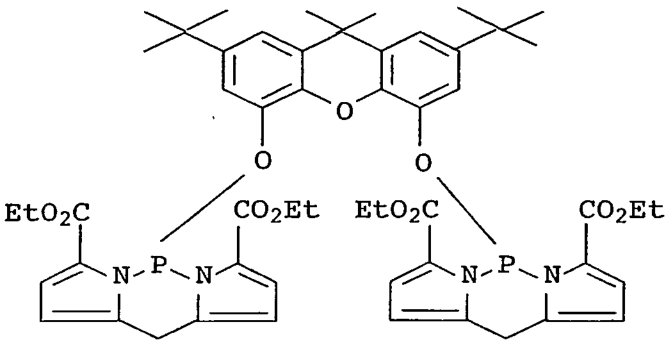

39

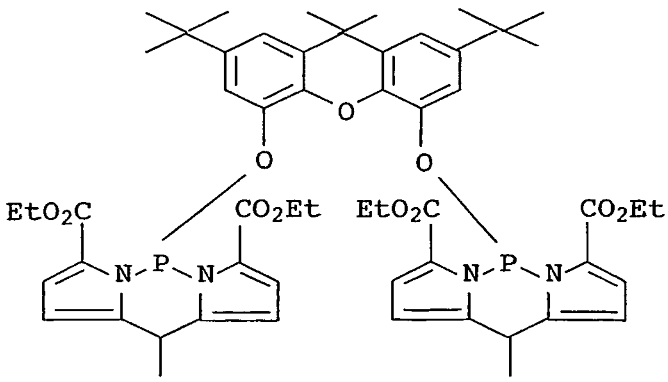

40

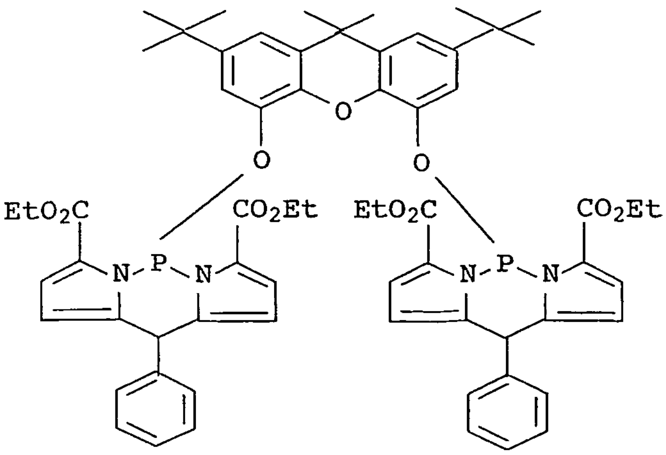

41

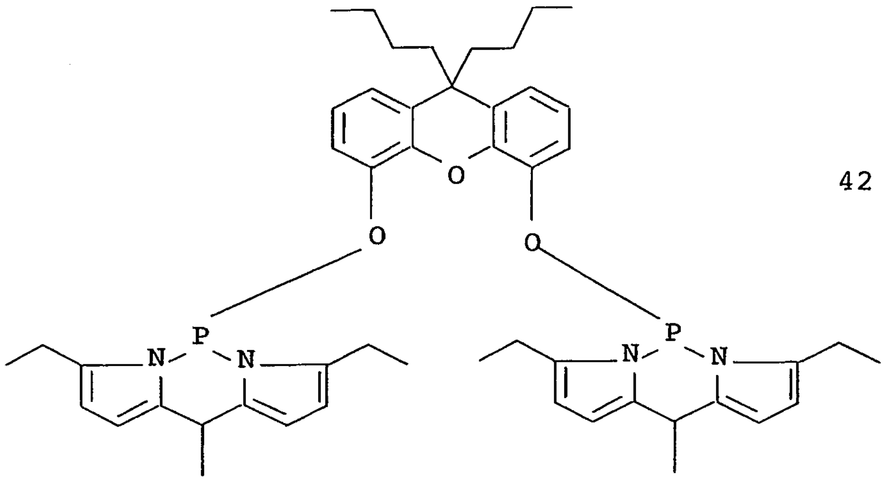

42

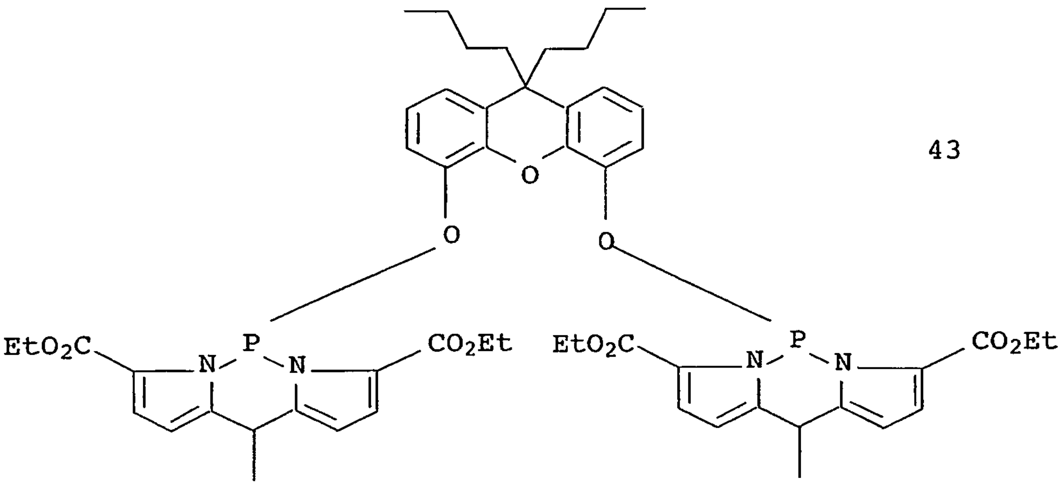

43

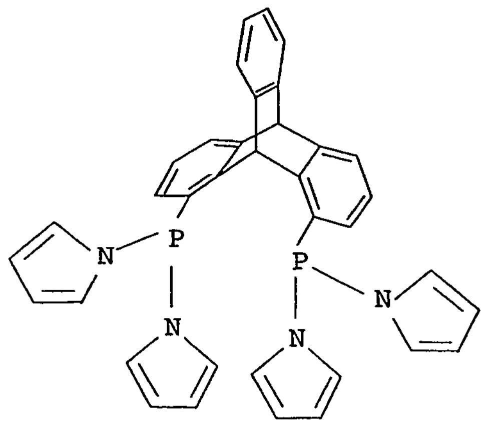

44

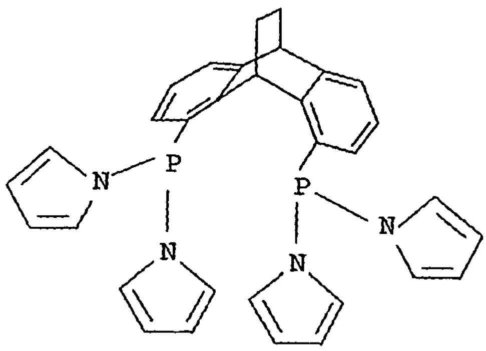

45

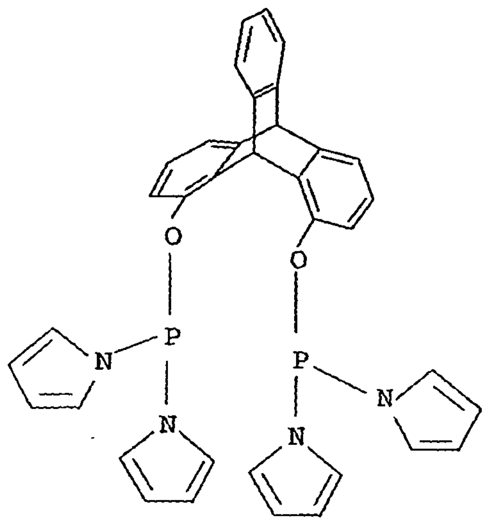

46

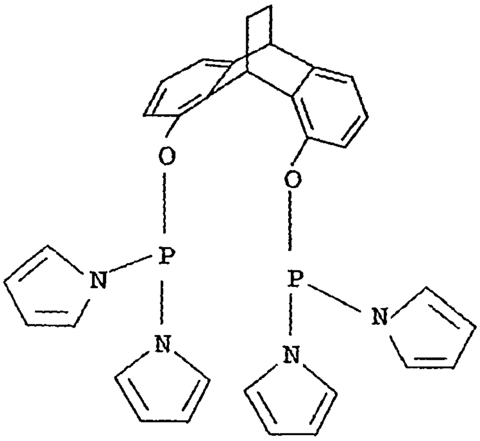

47

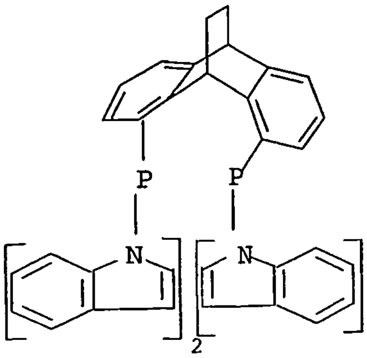

48

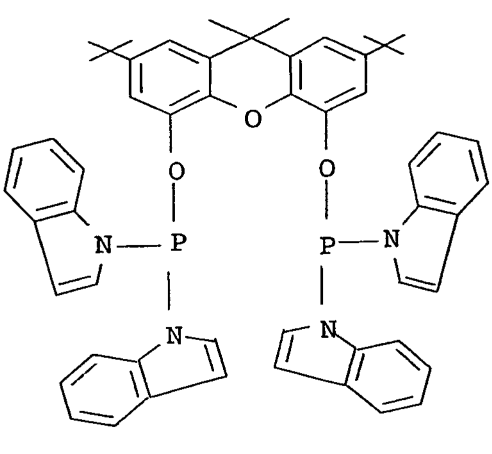

49 .

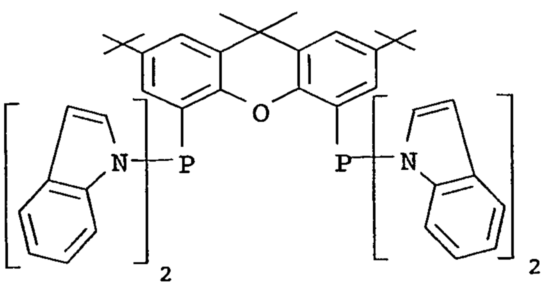

50 .

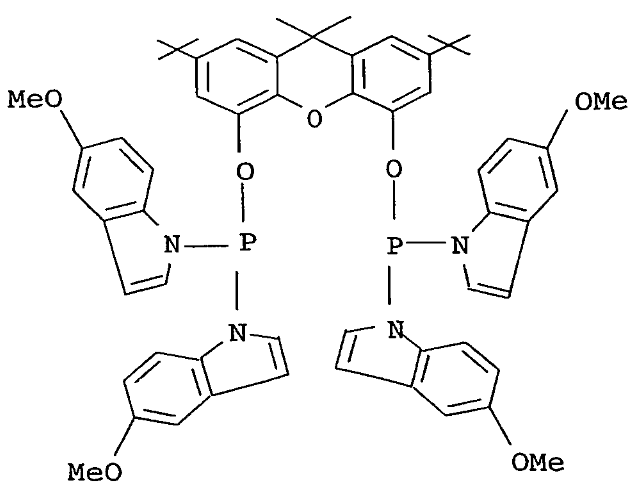

51

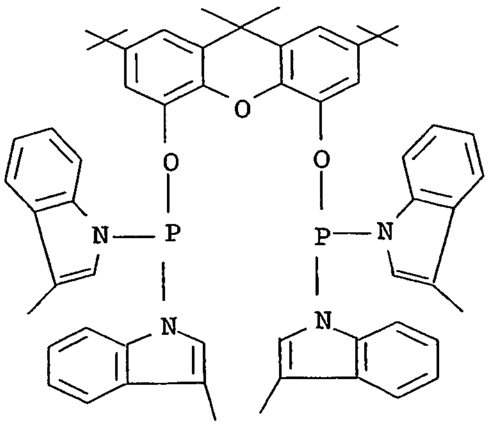

52

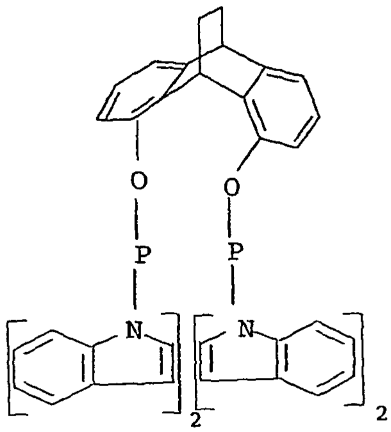

53

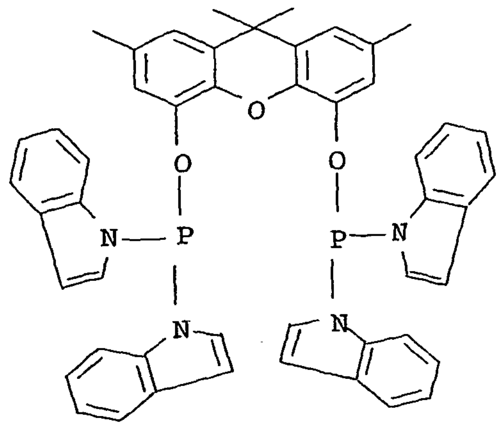

54

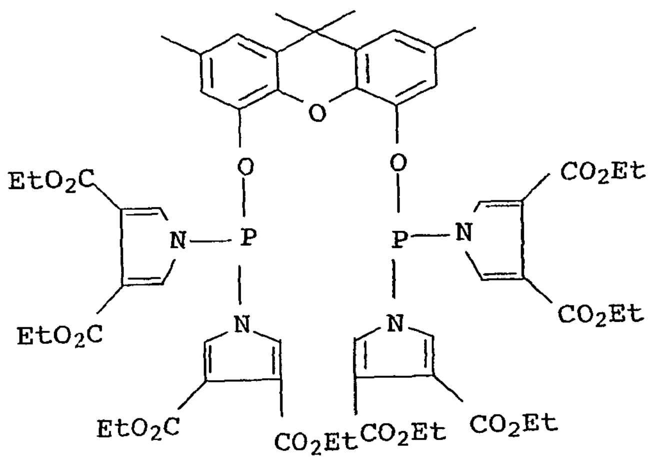

55

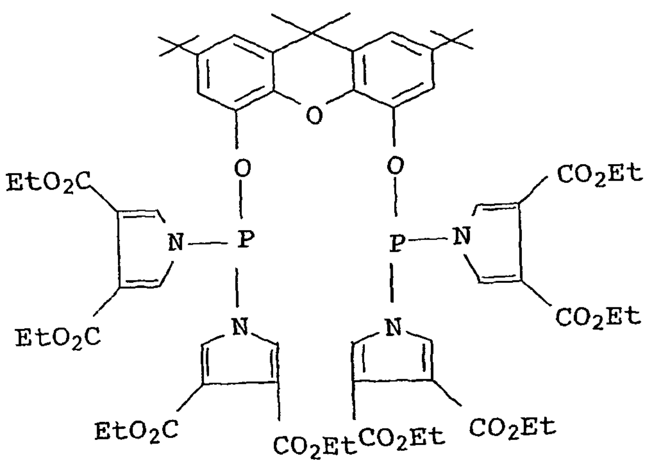

56

Me = Methyl
Et = Ethyl

Die Herstellung der Pnicogenchelatverbindungen ist in der WO 02/083695 beschrieben, auf die hiermit im vollen Umfang Bezug genommen wird.

Im Allgemeinen werden unter Hydroformylierungsbedingungen aus den jeweils eingesetzten Katalysatoren oder Katalysatorvorstufen katalytisch aktive Spezies der allgemeinen Formel $H_gZ_d(CO)_eG_f$ gebildet, worin Z für ein Metall der VIII. Nebengruppe, G für eine Phosphor-, Arsen- oder Antimon-haltigen Liganden der Formel I bzw. II und d, e, f, g für natürliche Zahlen, abhängig von der Wertigkeit und Art des Metalls sowie der Bindigkeit des Liganden G, stehen. Vorzugsweise stehen e und f unabhängig voneinander mindestens für einen Wert von 1, wie z. B. 1, 2 oder 3. Die Summe aus e und f steht bevorzugt für einen Wert von 2 bis 5. Dabei können die Komplexe des Metalls Z mit den erfindungsgemäß eingesetzten Liganden G gewünschtenfalls zusätzlich noch mindestens einen weiteren nicht erfindungsgemäß verwendeten Liganden, z. B. aus der Klasse der Triarylphosphine, insbesondere Triphenylphosphin, Triarylphosphite, Triarylphosphinite, Triarylphosphonite, Phosphabenzole, Trialkylphosphine oder Phosphametallocene enthalten. Derlei Komplexe des Metalls Z mit erfindungsgemäß verwendeten und nicht-erfindungsgemäß verwendeten Liganden bilden sich z. B. in einer Gleichgewichtsreaktion nach Zusatz eines Liganden zu einem Komplex der allgemeinen Formel $H_gZ_d(CO)_eG_f$.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die Katalysatoren des erfindungsgemäßen Verfahrens jedoch auch separat hergestellt und nach üblichen Verfahren isoliert werden. Zur in situ-Herstellung der Katalysatoren kann man wenigstens eine Verbindung der allgemeinen Formel I bzw. II, eine Verbindung oder einen Komplex eines Metalls der VIII. Nebengruppe, gewünschtenfalls einen oder mehrere weitere zusätzliche Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium (III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat (III) etc. Weiterhin eignen sich Rhodiumkomplexe, wie Rhodiumbiscarbonylacetylacetonat, Ace-tylacetonatobisethylenrhodium(I) etc. Vorzugsweise werden Rhodiumbiscarbonylacetylacetonat oder Rhodiumacetat eingesetzt. Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie $K_2RuO_4$ oder $KRuO_4$ oder Komplexverbindungen, wie z. B. $RuHCl(CO)(PPh_3)_3$. Auch können die Metallcarbonyle des Rutheniums wie Trisrutheniumdodecacarbonyl oder Hexarutheniumoctadecacarbonyl, oder Mischformen, in denen CO teilweise durch Liganden der Formel $PR_3$ ersetzt sind, wie $Ru(CO)_3(PPh_3)_2$, im erfindungsgemäßen Verfahren verwendet werden.

Geeignete Kobaltverbindungen sind beispielsweise Kobalt(II)chlorid, Kobalt(II)sulfat, Kobalt(II)carbonat, Kobalt (II)nitrat, deren Amin- oder Hydratkomplexe, Kobaltcarboxylate, wie Kobaltacetat, Kobaltethylhexanoat sowie Kobaltnaphthenoat. Auch hier können die Carbonylkomplexe des Kobalts wie Dikobaltoctacarbonyl, Tetrakobaltdodecacarbonyl und Hexakobalthexadecacarbonyl eingesetzt werden.

Die genannten und weitere geeignete Verbindungen des Kobalts, Rhodiums, Rutheniums und Iridiums sind

bekannt, kommerziell er-hältlich oder ihre Herstellung ist in der Literatur hinreichend beschrieben oder sie können vom Fachmann analog zu den bereits bekannten Verbindungen hergestellt werden.

Geeignete Metalle der VIII. Nebengruppe sind insbesondere Kobalt und Rhodium.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol®, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannte "Ionische Flüssigkeiten" verwendet werden. Hierbei handelt es sich um flüssige Salze, beispielsweise um N,N'-Dialkylimidazoliumsalze wie die N-Butyl-N'-methylimidazoliumsalze, Tetraalkylammoniumsalze wie die Tetran-butylammoniumsalze, N-Alkylpyridiniumsalze wie die n-Butylpyridiniumsalze, Tetraalkylphosphoniumsalze wie die Trishexyl(tetradecyl)phosphoniumsalze, z. B. die Tetrafluoroborate, Acetate, Tetrachloroaluminate, Hexafluorophosphate, Chloride und Tosylate.

Weiterhin ist es möglich die Umsetzungen auch in Wasser oder wässrigen Lösungsmittelsystemen, die neben Wasser ein mit Wasser mischbares Lösungsmittel, beispielsweise einen Alkohol wie Methanol, Ethanol, n-Propanol, Isopropanol, n-Butanol, Isobutanol, ein Keton wie Aceton und Methylethylketon oder ein anderes Lösungsmittel enthalten. Zu diesem Zweck setzt man Liganden der Formel I bzw. II ein, die mit polaren Gruppen, beispielsweise ionischen Gruppen wie $SO_3M$, $CO_2M$ mit M = Na, K oder $NH_4$ oder wie $N(CH_3)_4^+$ modifiziert sind. Die Umsetzungen erfolgen dann im Sinne einer Zweiphasenkatalyse, wobei der Katalysator sich in der wässrigen Phase befindet und Einsatzstoffe und Produkte die organische Phase bilden. Auch die Umsetzung in den "Ionischen Flüssigkeiten" kann als Zweiphasenkatalyse ausgestaltet sein.

1,9-Decadien wird großtechnisch von Shell hergestellt. 1,7-Octadien wird beispielsweise durch reduktive Kupplung von 1,3-Butadien in Gegenwart von Essigsäure und Triethylamin als Promotoren gewonnen.

Bevorzugt ist ein Verfahren, das dadurch gekennzeichnet ist, dass der Hydroformylierungskatalysator in situ hergestellt wird, wobei man mindestens eine Verbindung der Formel I bzw. II, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt. Gewünschtenfalls können die Ligand-Metall-Komplexe jedoch auch separat hergestellt und nach üblichen Verfahren isoliert werden.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Encyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Encyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 40 bis 80 °C, insbesondere etwa 50 bis 70 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten Hydroformylierungskatalysators variiert werden. Im Allgemeinen erlauben die Katalysatoren auf Basis von Phosphor-, Arsen- oder Antimon-haltigen Pnicogenchelat-Verbindungen eine Umsetzung in einem Bereich niedriger Drücke, wie etwa im Bereich von 1 bis 100 bar, bevorzugt 5 bis 50 bar.

Das Molmengenverhältnis von Pnicogenchelatverbindung I bzw. II zum Metall der VIII. Nebengruppe im Hydroformylierungsmedium liegt im Allgemeinen in einem Bereich von etwa 1:1 bis 1000:1, vorzugsweise von 1:1 bis 100:1, insbesondere von 1:1 bis 50:1 und ganz besonders bevorzugt 1:1 bis 20:1.

Üblicherweise liegt das molare Verhältnis von Metall der VIII. Nebengruppe zu Substrat unter 1 mol-%, vorzugsweise unter 0,5 mol-% und insbesondere unter 0,1 mol-% und ganz besonders bevorzugt unter 0,05 mol-%.

Die Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Die zuvor beschriebenen Katalysatoren können auch in geeigneter Weise, z. B. durch Anbindung über als Ankergruppen geeignete funktionelle Gruppen, Adsorption, Pfropfung, etc. an einen geeigneten Träger, z. B. aus Glas,

Kieselgel, Kunstharzen etc., immobilisiert werden. Sie eignen sich dann auch für einen Einsatz als Festphasenkatalysatoren.

Es hat sich gezeigt, dass ethylenisch ungesättigte Verbindungen, insbesondere solche mit wenigstens einer terminalen Doppelbindung, mit dem erfindungsgemäßen Verfahren bei niedrigen Temperaturen und niederen Drücken in vorteilhafter Weise hydroformyliert werden können. Dabei sind in der Regel kürzere Reaktionszeiten und/oder geringere Mengen an Katalysatorsystem, bezogen auf eingesetztes Substrat erforderlich, als zur Hydroformylierung des gleichen Substrats unter Verwendung des gleichen katalytisch aktiven Metalls mit anderen phosphorhaltigen Cokatalysatoren, wie Xantphos, erforderlich wären (siehe z. B. C. Botteghi et al. in J. Mol. Catal. A: Chem 2001, 175, 17, Tabelle 2: hohe Katalysatorbeladung von 0,4 - 1 mol%). Insbesondere ist mit dem erfindungsgemäßen Verfahren die Hydroformylierung ethylenisch ungesättigter Verbindungen, insbesondere solcher mit zwei terminalen Doppelbindungen, bei Reaktionszeiten von weniger als 15 h, vorzugsweise weniger als 10 h, bei geringen Einsatzmengen an Katalysatorsystem möglich. Vorteilhafterweise findet unter den Bedin-gungen der Hydroformylierung mit den erfindungsgemäß eingesetzten Katalysatoren keine oder nur in sehr geringem Maße Isomerisierung terminaler Doppelbindungen zu den thermodynamisch stabileren internen Doppelbindungn statt. Die eingesetzten Katalysatoren zeichnen sich somit durch eine hohe n-Selektivität aus, d. h. ausgehend von $\alpha,\omega$-Dioelfinen erhält man in hohen Ausbeuten $\alpha,\omega$-Enale und/oder $\alpha,\omega$-Dialdehyde.

Eine Ausführungsform der vorliegenden Erfindung betrifft die Herstellung von Dialdehyden. In einer bevorzugten Ausführungsform erfolgt die Herstellung der Dialdehyde diskontinuierlich. Diskontinuierliche Hydroformylierungsverfahren sind dem Fachmann prinzipiell bekannt. Nach beendeter Umsetzung entspannt man in der Regel zunächst den Reaktor. Das dabei freigesetzte Synthesegas und gegebenenfalls nicht umgesetzte, ungesättigte Verbindungen können - gegebenenfalls nach Aufarbeitung - ganz oder teilweise erneut eingesetzt werden. Der übrige Reaktorinhalt besteht im Wesentlichen aus Dialdehyd, hochsiedenden Nebenprodukten (im Folgenden auch als Hochsieder bezeichnet) und Katalysator. Zur Aufarbeitung kann der Reaktorinhalt einer ein- oder mehrstufigen Auftrennung unterworfen werden, wobei man zumindest eine an Dialdehyd angereicherte Fraktion erhält. Die Auftrennung in eine an Dialdehyd angereicherte Fraktion kann auf verschiedene Weise erfolgen, beispielsweise durch Destillation, Kristallisation oder Membramfiltration, vorzugsweise durch Destillation. In einer insbesonders bevorzugten Ausgestaltung des diskontinuierlichen Verfahrens verwendet man einen Reaktor mit aufgesetzter Destillationskolonne, so dass die Destillation direkt aus dem Reaktor erfolgen kann. Die Destillationskolonne ist gegebenenfalls mit Rektifikationsböden versehen, um eine möglichst gute Trennleistung zu erzielen. Die Destillation kann bei Normaldruck oder bei vermindertem Druck erfolgen. Am Kopf oder im oberen Bereich der Kolonne kann man die an Dialdehyd angereicherte Fraktion isolieren, wobei im Sumpf oder im unteren Bereich der Kolonne wenigstens eine an Dialdehyd abgereicherte Fraktion isoliert werden kann. Geeignete Kolonnen, Temperatur- und Druckparameter sind dem Fachmann bekannt. Die an Dialdehyd angereicherte Fraktion kann gegebenenfalls einem weiteren Reinigungsschritt unterzogen werden. Die an Dialdehyd abgereicherte Fraktion enthält im Wesentlichen Hochsieder sowie den Katalysator. Der Katalysator lässt sich nach üblichen, dem Fachmann bekannten Verfahren abtrennen und kann im Allgemeinen - gegebenenfalls nach Aufarbeitung - erneut in einer weiteren Hydroformylierung eingesetzt werden.

In einer weiteren bevorzugten Ausführungsform erfolgt die Herstellung der Dialdehyde kontinuierlich. Bei der kontinuierlichen Verfahrensführung unterwirft man eine ungesättigte Verbindung in einer oder mehreren Reaktionszonen der Hydroformylierung. Man entnimmt aus der Reaktionszone einen Austrag, der in der Regel zunächst entspannt wird. Dabei werden nicht umgesetztes Synthesegas sowie ungesättigte Verbindungen freigesetzt, die in der Regel - gegebenenfalls nach Aufarbeitung- in die Reaktionszone zurückgeführt werden. Die Auftrennung des verbleibenden Austrages in eine an Dialdehyd angereicherte Fraktion kann mittels üblicher, aus dem Stand der Technik bekannter Maßnahmen erfolgen, wie beispielsweise durch Destillation, Kristallisation oder Membranfiltration. Geeignete Destillationsanlagen sind dem Fachmann bekannt. Des Weiteren sind auch Dünnschichtverdampfer geeignet. Bei der destillativen Auftrennung entnimmt man aus dem Sumpf oder aus dem unteren Bereich der Kolonne eine im Wesentlichen aus Hochsiedern und Katalysator bestehende Fraktion, die direkt in die Reaktionszone zurückgeführt werden kann. Vorzugsweise schleust man die Hochsieder jedoch zuvor ganz oder teilweise vor der Rückführung aus und führt den Katalysator - gegebenenfalls nach Aufarbeitung - in die Reaktionszone zurück. Am Kopf oder im oberen Bereich der Kolonne entnimmt man wenigstens eine an Dialdehyd angereicherte Fraktion, die gegebenenfalls auch ungesättigten Monoaldehyd enthält. Zweckmäßigerweise unterzieht man die an Dialdehyd angereicherte, noch ungesättigten Monoaldehyd enthaltene Fraktion wenigstens einer weiteren Auftrennung, wobei man wenigstens eine an ungesättigtem Monoaldehyd angereicherte Fraktion und eine an Dialdehyd angereicherte Fraktion erhält. Die an ungesättigtem Monoaldehyd angereicherte Phase wird in die Reaktionszone zurückgeführt und die an Dialdehyd angereicherte Phase wird ausgeschleust.

In einer speziellen Ausführungsform betrifft die vorliegende Erfindung ein Verfahren zur Hydroformylierung von Verbindungen mit mindestens zwei ethylenisch ungesättigten Doppelbindungen unter Isolierung der darin gebildeten ungesättigten Monoaldehyde (Enale).

Ein weiterer Gegenstand der Erfindung ist ein daher Verfahren, bei dem man

(i) 1.9-Decadieu in einer Reaktionszone der Hydroformylierungsreaktion unterwirft,

(ii) aus der Reaktionszone einen Austrag entnimmt und in eine an ungesättigten Monoaldehyden angereicherte Fraktion und eine an ungesättigten Monoaldehyden abgereicherte Fraktion auftrennt, und

(iii) die gegebenenfalls aufgearbeitete, an ungesättigten Monoaldehyden abgereicherte Fraktion in die Reaktionszone zurück-führt.

Das Verfahren kann sowohl kontinuierlich, semikontinuierlich oder diskontinuierlich durchgeführt werden. Bevorzugt ist eine kontinuierliche Verfahrensführung.

In Schritt (i) des erfindungsgemäßen Verfahrens setzt man die wenigstens zwei ethylenisch ungesättigte Doppelbindungen enthaltende Verbindung mit Kohlenmonoxid und Wasserstoff in Gegenwart eines Hydroformylierungskatalysators, der wenigstens einen Komplex eines Metalls der VIII. Nebengruppe mit wenigstens einem Pnicogenchelat-Liganden der Formel I, wie zuvor beschrieben, enthält um. Bezüglich geeigneter und bevorzugter Hydroformylierungskatalysatoren und Reaktionsbedingungen wird auf das zuvor Gesagte Bezug genommen.

In Schritt (ii) entnimmt man aus der Reaktionszone einen Austrag, der im Wesentlichen unumgesetzte mehrfach ethylenisch ungesättigte Verbindungen, ungesättigten Monoaldehyd, Dialdehyd und Katalysator enthält. Die Hydroformylierungskatalysatoren lassen sich nach üblichen, dem Fachmann bekannten Verfahren abtrennen und können im Allgemeinen erneut für die Hydroformylierung eingesetzt werden. Die Auftrennung des in Schritt (i) erhaltenen Reaktionsgemisches in eine an ungesättigtem Monoaldehyd angereicherte und eine an ungesättigtem Monoaldehyd abgereicherte Fraktion kann mittels üblicher aus dem Stand der Technik bekannter Maßnahmen erfolgen (Schritt ii). Vorzugsweise erfolgt die Abtrennung destillativ, durch Kristallisation oder durch Membranfiltration.

Geeignete Destillationsanlagen umfassen alle dem Fachmann bekannten Destillationsvorrichtungen zur kontinuierlichen oder absatzweisen Auftrennung von flüssigen Stoffgemischen. Des Weiteren sind auch Dünnschichtverdampfer geeignet. Dazu zählen Vorrichtungen, in denen die aufzutrennenden Gemische durch Abrieselnlassen (Fallfilmverdampfer, Rieselkolonne), Zentrifugalkraft oder durch besonders konstruierte Wischer (Wischblattverdampfer, Sambay-Verdampfer, Filmtruder) auf beheizte Flächen verteilt werden.

Der Reaktionsaustrag aus der Reaktionszone wird vor einer destillativen Aufarbeitung in der Regel entspannt. Das dabei freigesetzte nicht umgesetzte Synthesegas und nicht umgesetzte Olefine können in die Reaktionszone zurückgeführt werden. Bei der destillativen Auftrennung wird die an ungesättigten Monoaldehyden angereicherte Fraktion im Allgemeinen als Kopfprodukt gewonnen. Die als Sumpfprodukt verbleibende an ungesättigten Monoaldehyden abgereicherte Fraktion kann gewünschtenfalls einer weiteren Auftrennung unter Erhalt einer an Katalysator angereicherten Fraktion und einer an Dialdehyd angereicherten Fraktion unterzogen werden. Die an Dialdehyd angereicherte Fraktion kann gewünschtenfalls als weiteres Wertprodukt ausgeschleust werden.

Das bei der destillativen Trennung rückgewonnene Substrat sowie das Katalysatorsystem werden in Schritt (iii) wieder in den Reaktor zurückgeführt und erneut der Hydroformylierung unterworfen.

Die Erfindung wird anhand der folgenden, nicht einschränkenden Beispiele näher erläutert.

Beispiele

Es wurde der folgende Ligand eingesetzt:

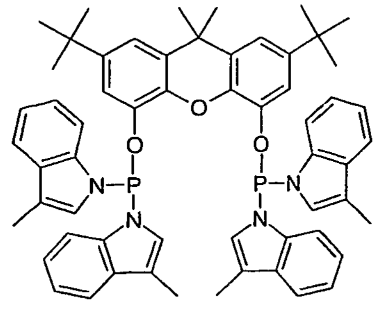

Ligand B

Beispiel 1:

Synthese von Ligand B

28,5 g (218 mmol) 3-Methylindol (Skatol) wurden bei Raumtemperatur in etwa 50 ml trockenem Toluol vorgelegt und das Lösungsmittel wurde im Vakuum abdestilliert (Entfernung von Wasserspuren). Dieser Vorgang wurde noch einnmal wiederholt. Der Rückstand wurde anschließend in 700 ml trockenem Toluol unter Argon aufgenommen und auf -65 °C abgekühlt. Anschließend wurden zunächst 14,9 g (109 mmol) $PCl_3$ und danach langsam 40 g (396 mmol) Triethylamin bei -65 °C zugegeben. Die Mischung wurde innerhalb 16 Stunden auf Raumtemperatur erwärmt und anschließend 16 h am Rückfluss erhitzt. Danach wurden 19,3 g (58 mmol) 4,5-Dihydroxy-2,7-di-tert-butyl-9,9-dimethylxanthen in 300 ml trockenem Toluol bei Raumtemperatur zugegeben und die Mischung wurde 16 h am Rückfluss erhitzt. Das entstandene Triethylaminhydrochlorid wurde abfiltriert und einmal mit Toluol nachgewaschen. Nach Einengen der organischen Phasen wurde der Rückstand zweimal aus heißem Ethanol umkristallisiert. Nach Trocknen im Vakuum wurden 36,3 g (71 % der Theorie) eines farblosen Feststoffes erhalten.
$^{31}$P-NMR (298 K) δ: 105 ppm.

Beispiel 2: Hydroformylierung von 1,9-Decadien

5,1 mg $Rh(CO)_2$acac und 202 mg Ligand B (100 ppm Rh = 0,03 mol%, Ligand:Rh = 11:1) wurden separat eingewogen, in je 5 g Toluol gelöst, vermischt und bei 100°C mit 10 bar Synthesegas ($CO:H_2$ = 1:1) begast. Nach 30 Minuten wurde auf 60°C abgekühlt, entspannt, dann wurden 10 g 1,9-Decadien zugegeben, 20 bar Synthesegas (CO: $H_2$ = 1:1) aufgepresst und 8 h bei 60°C hydroformyliert. Der Umsatz betrug 97%, die Dialselektivität 92% und die Linearität 98% (beide Doppelbindungen endständig hydroformyliert). Das erhaltene 1,12-Dodecandial wurde anschließend bei 130 bis 140 °C/7 bis 10 mbar (nicht geeicht) destilliert.
GC/MS (Ionisation: EI): Molpeak 198.
$^{1}$H-NMR ($CDCl_3$, 400 MHz, 298K): δ = 1,06 (breites s, C4, C4', C5, C5', C6, C6', 12H), 1,38 (Quintett, J = 7,1 Hz, C3, C3', 4H), 2,18 (dt, J = 1,7 Hz und 7,3 Hz, C2, C2', 4H), 9,50 (t, J = 1.7 Hz, C1, C1', 2H).
$^{13}C\{^{1}H\}$-NMR ($CDCl_3$, 101 MHz, 298K) [DEPT-135] δ = 22,18 (C6, C6', [$CH_2$]), 29,27 (C5, C5', [$CH_2$]), 29,47 (C4, C4', [$CH_2$]), 29,48 (C3, C3', [$CH_2$]), 43,92 (C2, C2', [$CH_2$]), 202,41 (C1, C1', [CH, $CH_3$]).

Beispiel 3: Reaktionskinetik: Bildung von Undec-10-en-1-al bei der Hydroformylierung von 1,9-Decadien

5,1 mg $Rh(CO)_2$acac und 202 mg Ligand B (100 ppm Rh, Ligand:Rh = 11:1) wurden separat eingewogen, in je 5 g Toluol gelöst, vermischt und bei 100°C mit 10 bar Synthesegas ($CO:H_2$ = 1:1) begast. Nach 30 Minuten wurde auf 60°C abgekühlt, entspannt, dann wurden 10 g 1,9-Decadien zugegeben, 20 bar Synthesegas ($CO:H_2$ = 1:1) aufgepresst und bei 60°C hydroformyliert . Es wurden nach verschiedenen Zeiten Proben entnommen und analysiert. Das Intermediat wurde zusätzlich durch GC-MS identifiziert. Figur 1 zeigt eine graphische Darstellung des Beispiels 5.

## Patentansprüche

1. Verfahren zur Herstellung von Dialdehyden und/oder ethylenisch ungesättigten Monoaldehyden durch Umsetzung (von 1,9-Decadion) mit Kohlenmonoxid und Wasserstoff in Gegenwart eines Hydroformylierungskatalysators, der wenigstens einen Komplex eines Metalls der VIII. Nebengruppe mit wenigstens einem Liganden umfasst, der ausgewählt ist unter Pnicogenchelatverbindungen der allgemeinen Formel I,

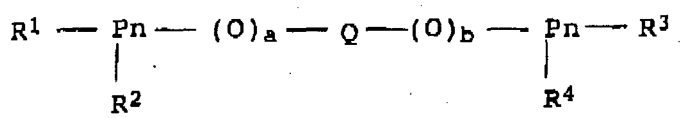

I

worin

Q eine Brückengruppe der Formel

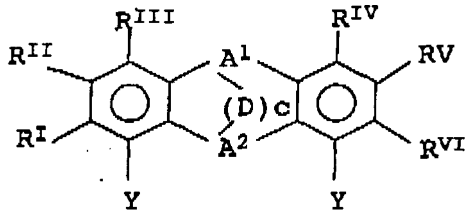

ist,
worin
$A^1$ und $A^2$ unabhängig voneinander für O, S, $SiR^aR^b$, $NR^c$ oder $CR^dR^e$ stehen, wobei
$R^a$,$R^b$ und $R^c$ unabhängig voneinander für Wasserstoff, Alkyl, Cycloalkyl, Heterocycloalkyl, Aryl oder Hetaryl stehen,
$R^d$ und $R^e$ unabhängig voneinander für Wasserstoff, Alkyl, Cycloalkyl, Heterocycloalkyl, Aryl oder Hetaryl stehen oder die Gruppe $R^d$ gemeinsam mit einer weiteren Gruppe $R^d$ oder die Gruppe $R^e$ gemeinsam mit einer weiteren Gruppe $R^e$ eine intramolekulare Brückengruppe D bilden, D eine zweibindige Brückengruppe, ausgewählt aus den Grup- pen

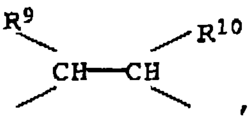 , 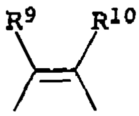 , 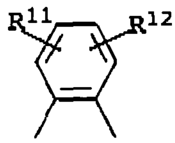 , 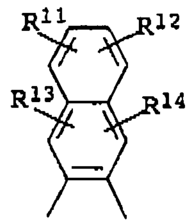

ist,
in denen
$R^9$ und $R^{10}$ unabhängig voneinander für Wasserstoff, Alkyl, Cycloalkyl, Aryl, Halogen, Trifluormethyl, Carboxyl, Carboxylat oder Cyano stehen oder miteinander zu einer $C_3$- bis $C_4$-Alkylenbrücke verbunden sind,
$R^{11}$, $R^{12}$, $R^{13}$ und $R^{14}$ unabhängig voneinander für Wasserstoff, Alkyl, Cycloalkyl, Aryl, Halogen, Trifluormethyl, COOH, Carboxylat, Cyano, Alkoxy, $SO_3H$, Sulfonat, $NE^1E^2$, Alky- len-$NE^1E^2E^{3+}X^-$, Acyl oder Nitro stehen,
c 0 oder 1 ist,
Y eine chemische Bindung darstellt,
$R^I$, $R^{II}$, $R^{III}$, $R^{IV}$, $R^V$ und $R^{VI}$ unabhängig voneinander für Was- serstoff, Alkyl, Cycloalkyl, Heterocycloalkyl, Aryl, Hetaryl, $COOR^f$, $COO^-M^+$, $SO_3R^f$, $SO^-{}_3M^+$, $NE^1E^2$, $NE^1E^2E^{3+}X^-$, Alkylen-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, Halogen, Trifluormethyl, Nitro, Acyl oder Cyano stehen,
worin
$R^f$, $E^1$, $E^2$ und $E^3$ jeweils gleiche oder verschiedene Reste, ausgewählt unter Wasserstoff, Alkyl, Cycloalkyl oder Aryl bedeuten,
$R^g$ für Wasserstoff, Methyl oder Ethyl steht,
$M^+$ für ein Kation steht,
$X^-$ für ein Anion steht, und
x oder für eine ganze Zahl von 1 bis 120 steht, zwei benachbarte Reste, ausgewählt unter $R^I$, $R^{II}$, $R^{III}$, $R^{IV}$, $R^V$ und $R^{VI}$ zusammen mit zwei benachbarten Kohlen- stoffatomen des Benzolkerns, an den sie gebunden sind, für ein kondensiertes Ringsystem, mit 1, 2 oder 3 wei- teren Ringen stehen,
a und b unabhängig voneinander die zahl 0 oder 1 bedeuten,
Pn für ein Pnicogenatom ausgewählt aus den Elementen und Phosphor, Arsen oder Antimon steht,
$R^1$, $R^2$, $R^3$, $R^4$ unabhängig voneinander für Hetaryl, Hetaryloxy, Alkyl, Alkoxy, Aryl, Aryloxy, Cycloalkyl, Cycloalkoxy, Heterocycloalkyl, Heterocycloalkoxy oder eine $NE^1E^2$-Gruppe stehen, mit der Maßgabe, dass $R^1$ und $R^3$ über das Stickstoffatom an das Pnicogenatom Pn gebundene Pyr- rolgruppen sind oder worin $R^1$ gemeinsam mit $R^2$ und/oder $R^3$ gemeinsam mit $R^4$ eine zweibindige Gruppe E der Formel

Py-I-W

bildet, worin

Py eine Pyrrolgruppe ist, die über das pyrrolische Stickstoffatom an das Pnicogenatom Pn gebunden ist,
I für eine chemische Bindung oder für O, S, $SiR^aR^b$, $NR^c$, gegebenenfalls substituiertes $C_1$-$C_{10}$-Alkylen oder $CR^hR^i$ steht,
W für Cycloalkyl, Cycloalkoxy, Aryl, Aryloxy, Hetaryl und oder Hetaryloxy steht,
$R^h$ und $R^i$ unabhängig voneinander für Wasserstoff, Alkyl, Cycloalkyl, Beterocycloalkyl, Aryl oder Hetaryl ste- hen, oder worin $R^1$ gemeinsam mit $R^2$ und/oder $R^3$ gemeinsam mit $R^4$ eine über die Stickstoffatome an das Pnicogenatom Pn gebundene Bispyrrolgruppe der Formel

Py-I-Py

bildet.

**2.** Verfahren nach Anspruch 1, bei dem man wenigstens einen Liganden der Formel I einsetzt, in dem die Reste $R^1$, $R^2$, $R^3$ und $R^4$ unabhängig voneinander ausgewählt sind unter Gruppen der Formeln I.a bis I.k

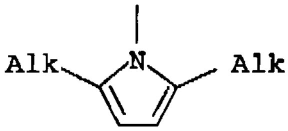

(I.a)

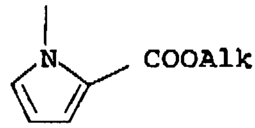

(I.b)

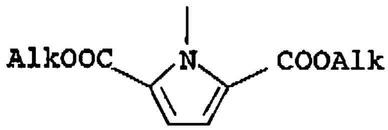

(I.c)

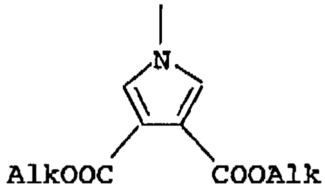

(I.d)

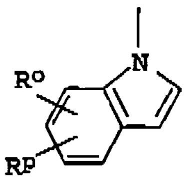

(I.e)

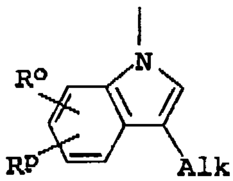

(I.f)

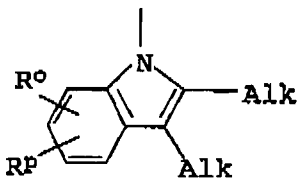

(I.g)

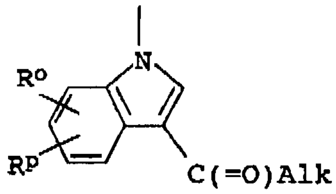

(I.h)

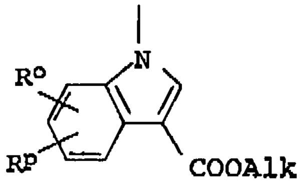

(I.i)

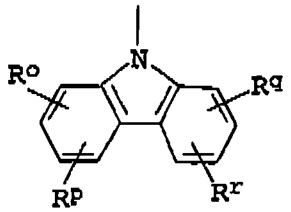

(I.k)

worin

Alk eine $C_1$-$C_4$-Alkylgruppe ist und
$R^o$, $R^p$, $R^q$ und $R^r$ unabhängig voneinander für Wasserstoff, $C_1$-$C_4$-Alkyl, $C_1$-$C_4$-Alkoxy, Acyl, Halogen, Trifluormethyl, $C_1$-$C_4$-Alkoxycarbonyl oder Carboxyl stehen.

**3.** Verfahren nach Anspruch 2, bei dem man wenigstens einen Liganden der Formel I einsetzt, in dem die Reste $R^1$, $R^2$, $R^3$ und $R^4$ unabhängig voneinander für eine 3-Alkylindolylgruppe, bevorzugt eine 3-Methylindolylgruppe, stehen.

**4.** Verfahren nach einem der vorherigen Ansprüche, wobei die Pnicogenchelatverbindung der Formel I ausgewählt ist unter Pnicogenchelatverbindung der allgemeinen Formel II,

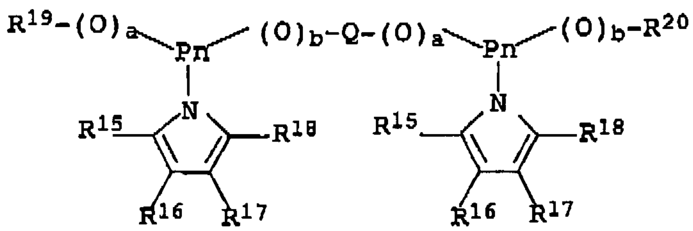

(II)

worin

$R^{15}$, $R^{16}$, $R^{17}$ und $R^{18}$ unabhängig voneinander für Wasserstoff, Alkyl, Cycloalkyl, Heterocycloalkyl, Aryl, Hetaryl, W'COOR$^k$, W'COO$^-$M$^+$, W'$(SO_3)$R$^k$, W'$(SO_3)^-$M$^+$, W'$PO_3$(R$^k$)(R$^1$), W'$(PO_3)^{2-}$$(M^+)_2$, W'NE$^4$E$^5$, W'(NE$^4$E$^5$E$^6$)$^+$X$^-$, W'OR$^k$, W'SR$^k$, $(CHR^1CH_2O)_yR^k$, $(CH_2NE^4)_yR^k$, $(CH_2CH_2NE^4)_yR^k$, Halogen, Tri- fluormethyl, Nitro, Acyl oder Cyano stehen, worin
W' für eine Einfachbindung, ein Heteroatom oder eine zweiwertige verbrückende Gruppe mit 1 bis 20 Brückenatomen steht,
$R^k$, $E^4$, $E^5$, $E^6$ jeweils gleiche oder verschiedene Reste, ausgewählt unter Wasserstoff, Alkyl, Cycloalkyl oder Aryl bedeuten,
$R^1$ für Wasserstoff, Methyl oder Ethyl steht,
$M^+$ für ein Kationäquivalent steht, f
y für eine ganze Zahl von 1 bis 240 steht,
mit wobei jeweils zwei benachbarte Reste $R^{15}$, $R^{16}$, $R^{17}$ und $R^{18}$ zu- sammen mit den Kohlenstoffatomen des Pyrrolrings, an die sie gebunden sind, auch für ein kondensiertes Ringsystem mit 1, 2 oder 3 weiteren Ringen stehen können, der Maßgabe, dass wenigstens einer der Reste $R^{15}$, $R^{16}$, $R^{17}$ oder $R^{18}$ nicht für Wasserstoff steht, und dass $R^{19}$ und $R^{20}$ nicht mit einander verknüpft sind,
$R^{19}$ und $R^{20}$ unabhängig voneinander für Cycloalkyl, Heterocy- cloalkyl, Aryl oder Hetaryl stehen, oder $R^{19}$ gemeinsam mit $R^{15}$ oder $R^{16}$ und/oder $R^{19}$ gemeinsam mit $R^{17}$ oder $R^{18}$ für eine zweibindinge Gruppe

-I-W-

stehen, worin

I für eine chemische Bindung oder für O, S, $SiR^aR^b$,$NR^c$ oder gegebenenfalls substituiertes $C_1$-$C_{10}$-Alkylen, bevorzugt $CR^hRi$, steht, worin $R^a$ , $R^b$, $R^c$, $R^h$ und $R^i$ unabhängig von- einander für Wasserstoff, Alkyl, Cycloalkyl, Heterocyclo- alkyl, Aryl oder Hetaryl stehen und

W für ycloalkyl, Cycloalkoxy, Aryl, Aryloxy, Hetaryl oder Hetaryloxy steht.

**5.** Verfahren nach einem der vorherigen Ansprüche, **dadurch gekennzeichnet, dass** die Pnicogenchelatverbindung der Formel I für eine Pnicogenchelatverbindung der allgemeinen Formel II.1 bis II.3 steht,

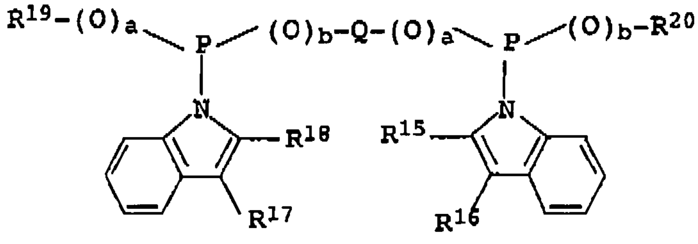

(II.1)

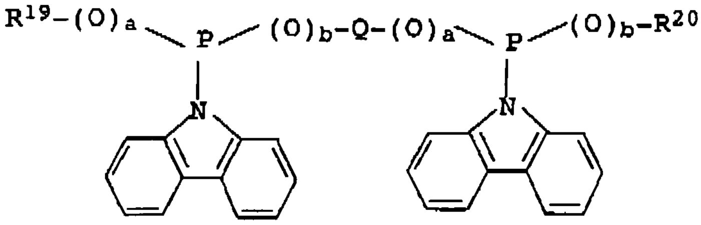

(II.2)

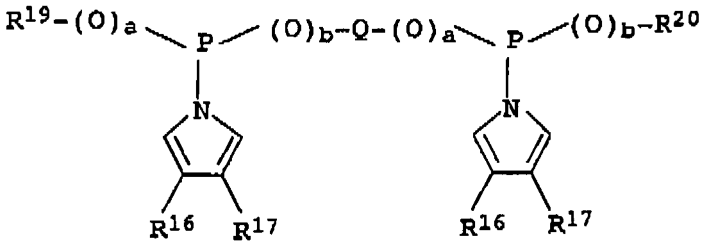

(II.3)

worin

$R^{15}$, $R^{16}$, $R^{17}$, $R^{18}$, Q, a und b die in Anspruch 4 angegebenen Bedeutungen besitzen, wobei in der Formel II. 3 wenigstens einer der Reste $R^{16}$ oder $R^{17}$ nicht für Wasserstoff steht,

$R^{19}$ und $R^{20}$ unabhängig voneinander für Cycloalkyl, Heterocy- cloalkyl, Aryl oder Hetaryl stehen.

**6.** Verfahren nach einem der Ansprüche 1 bis 5, wobei die Brückengruppe Q für eine Triptycendiyl-Gruppe der Formel

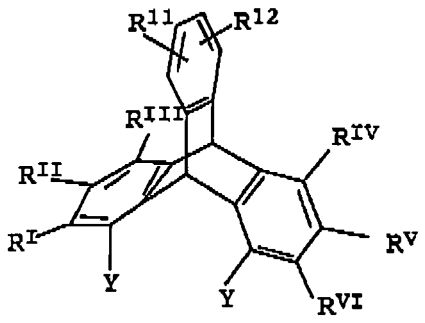

oder der Formel

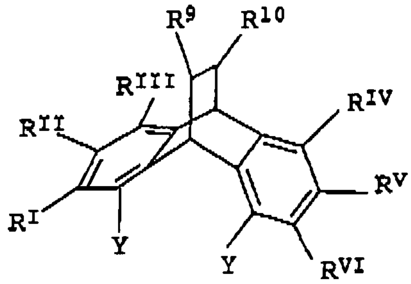

steht, in denen $R^I$, $R^{II}$, $R^{III}$, $R^{IV}$, $R^V$ und $R^{VI}$, $R^9$, $R^{10}$, $R^{11}$ und $R^{12}$ die in Anspruch 1 genannte Bedeutung haben.

7. Verfahren nach einem der Ansprüche 1 bis 5, **dadurch gekennzeichnet, dass** die Brückengruppe Q für eine Xanthendiyl-Gruppe der Formel

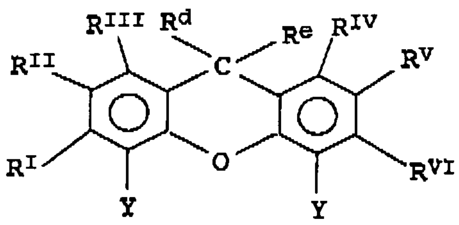

steht, in der $R^I$, $R^{II}$, $R^{III}$, $R^{IV}$, RV und $R^{VI}$ und Y die in Anspruch 1 genannte Bedeutung haben und $R^d$ und $R^e$ unabhängig voneinander für Wasserstoff, Alkyl, Cycloalkyl, Heterocyloalkyl, Aryl oder Hetaryl stehen.

8. Verfahren nach einem der vorherigen Ansprüche, wobei man in der Reaktionsmischung ein molares Verhältnis von Ligand zu Metall der VIII. Nebengruppe von 1:1 bis 1000:1 einstellt.

9. Verfahren nach einem der vorherigen Ansprüche, wobei man die Umsetzung bei Temperaturen im Bereich von 40 bis 80 °C durchführt.

10. Verfahren nach einem der vorherigen Ansprüche, **dadurch gekennzeichnet, dass** man

(i) 1,9-Decadien in einer Reaktionszone der Hydroformylierungsreaktion unterwirft,
(ii)aus der Reaktionszone einen Austrag entnimmt und in eine an ungesättigten Monoaldehyden angereicherte Fraktion und eine an ungesättigten Monoaldehyden abgereicherte Fraktion auftrennt, und
(iii)die gegebenenfalls aufgearbeitete, an ungesättigten Monoaldehyden abgereicherte Fraktion in die Reaktionszone zurückführt.

## Claims

**1.** A process for preparing dialdehydes and/or ethylenically unsaturated monoaldehydes by reacting 1,9-decadiene with carbon monoxide and hydrogen in the presence of a hydroformylation catalyst comprising at least one complex of a metal of transition group VIII with at least one ligand selected from among chelating pnicogen compounds of the formula I,

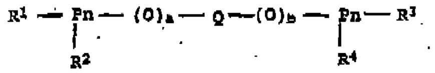 I

where

Q is a bridging group of the formula

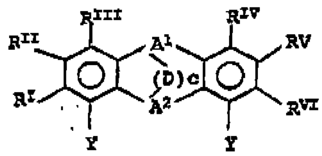

where
$A^1$ and $A^2$ are each, independently of one another, O, S, $SiR^aR^b$, $NR^c$ or $CR^dR^e$, where
$R^a$,$R^b$ and $R^c$ are each, independently of one another, hydrogen, alkyl, cycloalkyl, heterocycloalkyl, aryl or hetaryl,
$R^d$ and $R^e$ are each, independently of one another, hydrogen, alkyl, cycloalkyl, heterocycloalkyl, aryl or hetaryl or the group $R^d$ together with a further group $R^d$ or the group $R^e$ together with a further group $R^e$ form an intramolecular bridging group D,
D is a divalent bridging group selected from among the groups

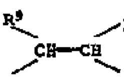 , 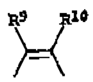 , 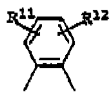 , 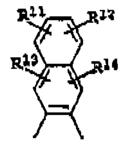

where
$R^9$ and $R^{10}$ are each, independently of one another, hydrogen, alkyl, cycloalkyl, aryl, halogen, trifluoromethyl, carboxyl, carboxylate or cyano or are joined to one another to form a $C_3$-$C_4$- alkylene bridge,
$R^{11}$, $R^{12}$, $R^{13}$ and $R^{14}$ are each, independently of one another, hydrogen, alkyl, cycloalkyl, aryl, halogen, trifluoromethyl, COOH, carboxylate, cyano, alkoxy, $SO_3H$, sulfonate, $NE^1E^2$, alkylene- $NE^1E^2E^{3+}X^-$, acyl or nitro,
c is 0 or 1,
Y is a chemical bond,
$R^I$, $R^{II}$, $R^{III}$, $R^{IV}$, $R^V$ and $R^{VI}$ are each, independently of one another, hydrogen, alkyl, cycloalkyl, heterocycloalkyl, aryl, hetaryl, $COOR^f$, $COO^-M^+$, $SO_3R^f$, $SO^-{}_3M^+$, $NE^1E^2$, $NR^1E^2E^{3+}X^-$, alkylene-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, halogen, trifluoromethyl, nitro, acyl or cyano, where
$R^f$, $E^1$, $E^2$ and $E^3$ are identical or different radicals selected from among hydrogen, alkyl, cycloalkyl and aryl,
$R^g$ is hydrogen, methyl or ethyl,
$M^+$ is a cation,
$X^-$ is an anion, and
x is an integer from 1 to 120,
or two adjacent radicals selected from among $R^I$, $R^{II}$, $R^{III}$, $R^{IV}$, $R^V$ and $R^{VI}$ together with two adjacent carbon atoms of the benzene ring to which they are bound form a fused ring system having 1, 2 or 3 further rings,
a and b are each, independently of one another, 0
or 1, Pn is a pnicogen atom selected from among the elements phosphorus, arsenic and antimony,
and

$R^1$, $R^2$, $R^3$, $R^4$ are each, independently of one another, hetaryl, hetaryloxy, alkyl, alkoxy, aryl, aryloxy, cycloalkyl, cycloalkoxy, heterocycloalkyl, heterocycloalkoxy or an $NE^1E^2$ group, with the proviso that $R^1$ and $R^3$ are pyrrole groups bound via the nitrogen atom to the pnicogen atom Pn
or $R^1$ together with $R^2$ and/or $R^3$ together with $R^4$ form a divalent group E of the formula

Py-I-W

where
Py is a pyrrole group which is bound via the pyrrole nitrogen atom to the pnicogen atom Pn,
I is a chemical bond or O, S, $SiR^aR^b$, $NR^c$, substituted or unsubstituted $C_1$-$C_{10}$- alkylene or $CR^hR^i$,
W is cycloalkyl, cycloalkoxy, aryl, aryloxy, hetaryl or hetaryloxy,
and
$R^h$ and $R^i$ are each, independently of one another, hydrogen, alkyl, cycloalkyl, heterocycloalkyl, aryl or hetaryl,

or $R^1$ together with $R^2$ and/or $R^3$ together with $R^4$ form a bispyrrole group of the formula

Py-I-Py

bound via the nitrogen atoms to the pnicogen atom Pn.

**2.** The process according to claim 1, wherein at least one ligand of the formula I, in which the radicals $R^1$, $R^2$, $R^3$ and $R^4$ are selected independently from among groups of the formulae I.a to I.k

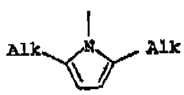
(I.a)

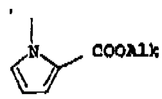
(I.b)

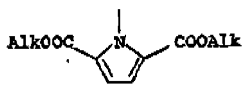
(I.c)

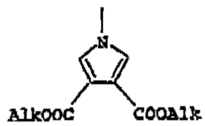
(I.d)

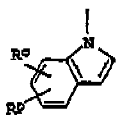
(I.e)

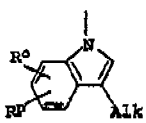
(I.f)

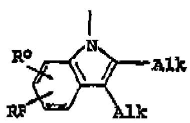
(I.g)

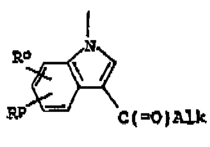
(I.h)

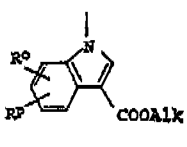
(I.i)

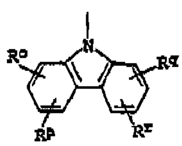
(I.k)

where

Alk is a $C_1$-$C_4$-alkyl group and
$R^o$, $R^p$, $R^q$ and $R^r$ are each, independently of one another, hydrogen, $C_1$-$C_4$-alkyl, $C_1$-$C_4$-alkoxy, acyl, halogen, trifluoromethyl, $C_1$-$C_4$- alkoxycarbonyl or carboxyl, is used.

**3.** The process according to claim 2, wherein at least one ligand of the formula I, in which the radicals $R^1$, $R^2$, $R^3$ and $R^4$ are each, independently of one another, a 3-alkylindolyl group, preferably a 3-methylindolyl group, is used.

**4.** The process according to any of the preceding claims, wherein the chelating pnicogen compound of the formula I is selected from among chelating pnicogen compounds of the formula II,

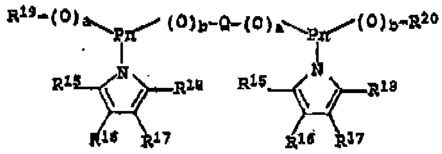

(II)

where

$R^{15}$, $R^{16}$, $R^{17}$ and $R^{18}$ are each, independently of one another, hydrogen, alkyl, cycloalkyl, heterocycloalkyl, aryl, hetaryl, $W'COOR^k$, $W'COO^- M^+$, $W'(SO_3)R^k$, $W'(SO_3)^-M^+$, $W'PO_3(R^k)(R^1)$, $W'(PO_3)^{2-}(M^+)_2$, $W'NE^4E^5$, $W'(NE^4E^5E^6)^+X^-$, $W'OR^k$, $W'SR^k$, $(CHR^1CH_2O)_yR^k$, $(CH_2NE^4)_yR^k$, $(CH_2CH_2NE^4)_yR^k$, halogen, trifluoromethyl, nitro, acyl or cyano, where

W' is a single bond, a heteroatom or a divalent bridging group having from 1 to 20 bridge atoms,

$R^k$, $E^4$, $E^5$, $E^6$ are identical or different radicals selected from among hydrogen, alkyl, cycloalkyl and aryl,
$R^1$ is hydrogen, methyl or ethyl,
$M^+$ is a cation equivalent,
$X^-$ is an anion equivalent and
y is an integer from 1 to 240,
where two adjacent radicals $R^{15}$, $R^{16}$, $R^{17}$ and $R^{18}$ together with the carbon atoms of the pyrrole ring to which they are bound may also form a fused ring system having 1, 2 or 3 further rings,
with the proviso that at least one of the radicals $R^{15}$, $R^{16}$, $R^{17}$ and $R^{18}$ is not hydrogen and $R^{19}$ and $R^{20}$ are not joined to one another,
$R^{19}$ and $R^{20}$ are each, independently of one another, cycloalkyl, heterocycloalkyl, aryl or hetaryl, or $R^{19}$ together with $R^{15}$ or $R^{16}$ and/or $R^{19}$ together with $R^{17}$ or $R^{18}$ form a divalent group

-I-W-

where
I is a chemical bond or O, S, $SiR^aR^b$, $NR^c$ or substituted or unsubstituted $C_1$-$C_{10}$-alkylene, preferably $CR^hR^i$, where $R^a$, $R^b$, $R^c$, $R^h$ and $R^i$ are each, independently of one another, hydrogen, alkyl, cycloalkyl, heterocycloalkyl, aryl or hetaryl and
W is cycloalkyl, cycloalkoxy, aryl, aryloxy, hetaryl or hetaryloxy.

**5.** The process according to any of the preceding claims, wherein the chelating pnicogen compound of the formula I is a chelating pnicogen compound of the formulae II.1 to II.3,

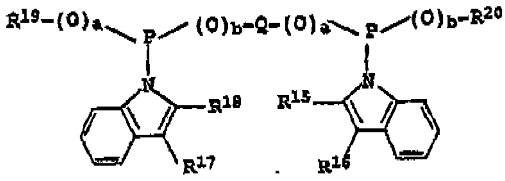

(II.1)

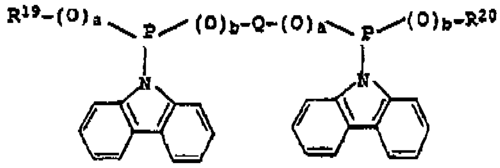

(II.2)

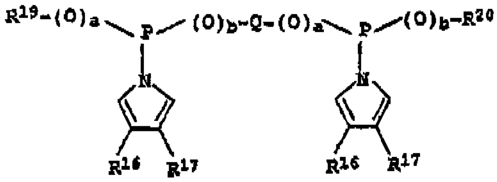

(II.3)

where

$R^{15}$, $R^{16}$, $R^{17}$, $R^{18}$, Q, a and b are as defined in claim 4, where at least one of the radicals $R^{16}$ and $R^{17}$ in the formula II.3 is not hydrogen,
$R^{19}$ and $R^{20}$ are each, independently of one another, cycloalkyl, heterocycloalkyl, aryl or hetaryl.

**6.** The process according to any of claims 1 to 5, wherein the bridging group Q is a triptycenediyl group of the formula

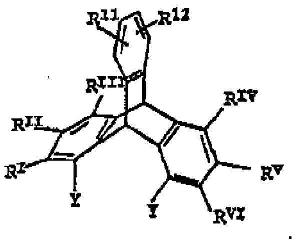

or the formula

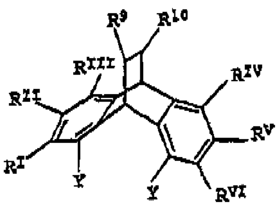

where $R^I$, $R^{II}$, $R^{III}$, $R^{IV}$, $R^V$ and $R^{VI}$, $R^9$, $R^{10}$, $R^{11}$ and $R^{12}$ are as defined in claim 1.

7. The process according to any of claims 1 to 5, wherein the bridging group Q is a xanthenediyl group of the formula

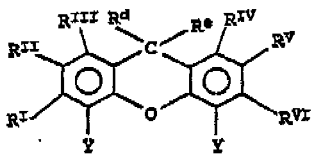

where $R^I$, $R^{II}$, $R^{III}$, $R^{IV}$, $R^V$ and $R^{VI}$ and Y are as defined in claim 1 and $R^d$ and $R^e$ are each, independently of one another, hydrogen, alkyl, cycloalkyl, heterocyloalkyl, aryl or hetaryl.

8. The process according to any of the preceding claims, wherein a molar ratio of ligand to metal of transition group VIII of from 1:1 to 1000:1 is set in the reaction mixture.

9. The process according to any of the preceding claims, wherein the reaction is carried out at from 40 to 80°C.

10. The process according to any of the preceding claims, wherein

(i) 1,9-decadiene is subjected to the hydroformylation reaction in a reaction zone,
(ii) an output is taken from the reaction zone and is fractionated to give a fraction enriched in unsaturated monoaldehydes and a fraction depleted in unsaturated monoaldehydes, and
(iii) the fraction depleted in unsaturated monoaldehydes is recirculated, if appropriate after work-up, to the reaction zone.

## Revendications

1. Procédé pour la préparation de dialdéhydes et/ou de monoaldéhydes à insaturation éthylénique, par mise en réaction de 1,9-décadiène avec du monoxyde de carbone et de l'hydrogène en présence d'un catalyseur d'hydroformylation qui comprend au moins un complexe d'un métal du groupe VIIIB avec au moins un ligand qui est choisi parmi des composés chélate de pnictogène de formule générale I,

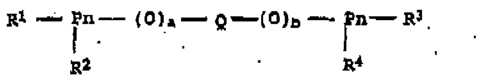 I

dans laquelle

Q est un groupe pontant de formule

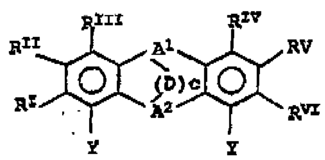

dans laquelle
$A^1$ et $A^2$ représentent, indépendamment l'un de l'autre, O, S, $SiR^aR^b$, $NP^c$ ou $CR^dR^e$,
$R^a$, $R^b$ et $R^c$ représentant chacun indépendamment un atome d'hydrogène, un groupe alkyle, cycloalkyle, hétérocycloalkyle, aryle ou hétéroaryle,
$R^d$ et $R^e$ représentent, indépendamment l'un de l'autre, un atome d'hydrogène, un groupe alkyle, cycloalkyle, hétérocycloalkyle, aryle ou hétéroaryle, ou le groupe $R^d$ conjointement avec un autre groupe $R^d$ ou le groupe $R^e$ conjointement avec un autre groupe $R^e$ forment un groupe pontant intramoléculaire D,

D est un groupe pontant à deux liaisons, choisi parmi les groupes

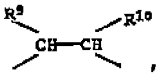 , 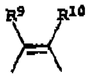 , 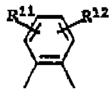 , 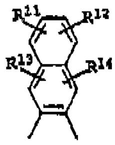

dans lesquels
$R^9$ et $R^{10}$ représentent, indépendamment l'un de l'autre, un atome d'hydrogène ou d'halogène ou un groupe alkyle, cycloalkyle, aryle, trifluorométhyle, carboxy, carboxylate ou cyano ou sont liés l'un à l'autre en un pont alkylène en $C_3$-$C_4$,
$R^{11}$, $R^{12}$, $R^{13}$ et $R^{14}$ représentent, indépendamment les uns des autres, un atome d'hydrogène ou d'halogène ou un groupe alkyle, cycloalkyle, aryle, trifluorométhyle, COOH, carboxylate, cyano, alcoxy, $SO_3H$, sulfonate, $NE^1E^2$, alkylène-$NE^1E^2E^{3+}X^-$, acyle ou nitro,
c est 0 ou 1,
Y représente une liaison chimique,
$R^I$, $R^{II}$, $R^{III}$, $R^{IV}$, $R^V$ et $R^{VI}$ représentent, indépendamment les uns des autres, un atome d'hydrogène, un groupe alkyle, cycloalkyle, hétérocycloalkyle, aryle, hétéroaryle, $COOR^f$, $COO^-M^+$, $SO_3R^f$, $SO^-_3M^+$, $NE^1E^2$, $NE^1E^2E^{3+}X^-$, alkylène-$NE^1E^2E^{3+}X^-$, $OR^f$, $SR^f$, $(CHR^gCH_2O)_xR^f$, $(CH_2N(E^1))_xR^f$, $(CH_2CH_2N(E^1))_xR^f$, un atome d'halogène, un groupe trifluorométhyle, nitro, acyle ou cyano,
où
$R^f$, $E^1$, $E^2$ et $E^3$ représentent chacun des radicaux identiques ou différents, choisis parmi un atome d'hydrogène, un groupe alkyle, cycloalkyle ou aryle,
$R^g$ représente un atome d'hydrogène, le groupe méthyle ou éthyle,
$M^+$ représente un cation,
$X^-$ représente un anion, et
x représente un nombre entier allant de 1 à 120,
ou deux radicaux contigus, choisis parmi $R^I$, $R^{II}$, $R^{III}$, $R^{IV}$, $R^V$ et $R^{VI}$ représentent ensemble, avec deux atomes de carbone contigus du noyau benzénique, auxquels ils sont liés, un système cyclique condensé, comportant 1, 2 ou 3 autres cycles,
a et b représentent, indépendamment l'un de l'autre, le nombre 0 ou 1,
Pn représente un atome de pnictogène choisi parmi les éléments phosphore, arsenic et antimoine,
et
$R^1$, $R^2$, $R^3$, $R^4$ représentent, indépendamment l'un de l'autre, un groupe hétéroaryle, hétéroaryloxy, alkyle, alcoxy, aryle, aryloxy, cycloalkyle, cycloalcoxy, hétérocycloalkyle, hétérocycloalcoxy ou un groupe $NE^1E^2$, étant entendu que $R^1$ et $R^3$ sont des groupes pyrrole liés par l'atome d'azote à l'atome de pnictogène Pn ou dans lesquels $R^1$ forme conjointement avec $R^2$ et/ou $R^3$ forme conjointement avec $R^4$ un groupe E à deux liaisons de formule

Py-I-W

dans laquelle
Py est un groupe pyrrole qui est lié à l'atome de pnictogène Pn par l'atome d'azote du groupe pyrrole,
I représente une liaison chimique ou O, S, $SiR^aR^b$, $NR^c$, un groupe alkylène en $C_1$-$C_{10}$ éventuellement substitué ou $CR^hR^i$,
W représente un groupe cycloalkyle, cycloalcoxy, aryle, aryloxy, hétéroaryle ou hétéroaryloxy,

et

$R^h$ et $R^i$ représentent, indépendamment l'un de l'autre, un atome d'hydrogène, un groupe alkyle, cycloalkyle, hétérocycloalkyle, aryle ou hétéroaryle, ou dans laquelle $R^1$ forme conjointement avec $R^2$ et/ou $R^3$ forme conjointement avec $R^4$ un groupe bispyrrole, lié à l'atome de pnictogène Pn par les atomes d'azote, de formule

Py-I-Py.

**2.** Procédé selon la revendication 1, dans lequel on utilise au moins un ligand de formule I, dans lequel les radicaux $R^1$, $R^2$, $R^3$ et $R^4$ sont choisis, indépendamment les uns des autres, parmi les groupes de formules I.a à I.k

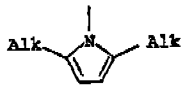

(I.a)

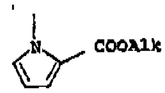

(I.b)

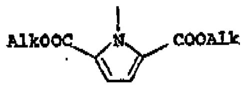

(I.c)

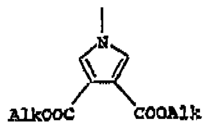

(I.d)

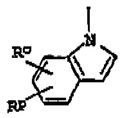

(I.e)

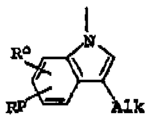

(I.f)

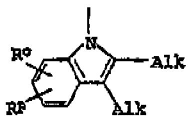

(I.g)

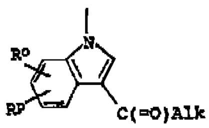

(I.h)

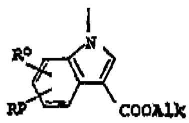

(I.i)

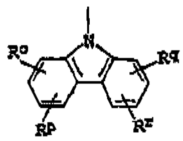

(I.k)

dans lesquelles

Alk est un groupe alkyle en $C_1$-$C_4$ et
$R^o$, $R^p$, $R^q$ et $R^r$ représentent, indépendamment l'un de l'autre, un atome d'hydrogène ou d'halogène ou un groupe alkyle en $C_1$-$C_4$, alcoxy en $C_1$-$C_4$, acyle, trifluorométhyle, alcoxy($C_1$-$C_4$)carbonyle ou carboxy.

**3.** Procédé selon la revendication 2, dans lequel on utilise au moins un ligand de formule I dans lequel les radicaux $R^1$, $R^2$, $R^3$ et $R^4$ représentent, indépendamment les uns des autres, un groupe 3-alkylindolyle, de préférence un groupe 3-méthyl-indolyle.

**4.** Procédé selon l'une quelconque des revendications précédentes, dans lequel le composé chélate de pnictogène de formule I est choisi parmi des composés chélate de pnictogène de formule générale II,

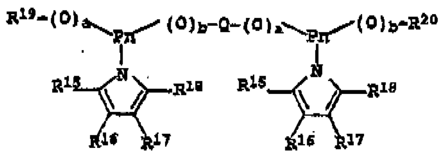

(II)

dans laquelle

$R^{15}$, $R^{16}$, $R^{17}$ et $R^{18}$ représentent, indépendamment les uns des autres, un atome d'hydrogène ou d'halogène ou un groupe alkyle, cycloalkyle, hétérocycloalkyle, aryle, hétéroaryle, $W'COOR^k$, $W'COO^-M^+$, $W'(SO_3)R^k$, $W'(SO_3)^-M^+$, $W'PO_3(R^k)(R^1)$, $W'(PO_3)^{2-}(M^+)_2$, $W'NE^4E^5$, $W'(NE^4E^5E^6)^+X^-$, $W'OR^k$, $W'SR^k$, $(CHR^1CH_2O)_yR^k$, $(CH_2NE^4)_yR^k$, $(CH_2CH_2NE^4)_yR^k$, trifluorométhyle, nitro, acyle ou cyano, où
W' représente une liaison simple, un hétéroatome ou un groupe pontant divalent ayant de 1 à 20 atomes formant le pont,
$R^k$, $E^4$, $E^5$, $E^6$ représentent chacun des radicaux identiques ou différents, choisis parmi un atome d'hydrogène, un groupe alkyle, cycloalkyle ou aryle,
$R^1$ représente un atome d'hydrogène, le groupe méthyle ou éthyle,
$M^+$ représente un équivalent d'un cation,
$X^-$ représente un équivalent d'un anion et
y représente un nombre entier allant de 1 à 240, chaque fois deux radicaux parmi $R^{15}$, $R^{16}$, $R^{17}$ et $R^{18}$ contigus pouvant également représenter, conjointement avec les atomes de carbone du cycle pyrrole auxquels ils sont liés, un système cyclique condensé comportant 1, 2 ou 3 autres cycles, étant entendu qu'au moins l'un des radicaux $R^{15}$, $R^{16}$, $R^{17}$ et $R^{18}$ ne représente pas un atome d'hydrogène et que $R^{19}$ et $R^{20}$ ne sont pas liés l'un à l'autre,
$R^{19}$ et $R^{20}$ représentent, indépendamment l'un de l'autre, un groupe cycloalkyle, hétérocycloalkyle, aryle ou hétéroaryle, ou $R^{19}$ forme conjointement avec $R^{15}$ ou $R^{16}$ et/ou $R^{19}$ forme conjointement avec $R^{17}$ ou $R^{18}$ un groupe à deux liaisons

-I-W-

dans laquelle
1 représente une liaison chimique ou représente O, S, $SiR^aR^b$, $NR^c$ ou un groupe alkylène en $C_1$-$C_{10}$ éventuellement substitué, de préférence $CR^hR^i$, $R^a$, $R^b$, $R^c$, $R^h$ et $R^i$ représentant, indépendamment les uns des autres, un atome d'hydrogène ou un groupe alkyle, cycloalkyle, hétérocycloalkyle, aryle ou hétéroaryle et
W représente un groupe cycloalkyle, cycloalcoxy, aryle, aryloxy, hétéroaryle ou hétéroaryloxy.

5. Procédé selon une ou plusieurs des revendications précédentes, **caractérisée en ce que** le composé chélate de pnictogène de formule I représente un composé chélate de pnictogène de formules générales II.1 à II.3,

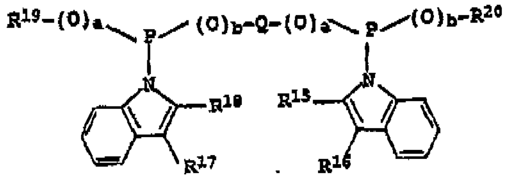

(II.1)

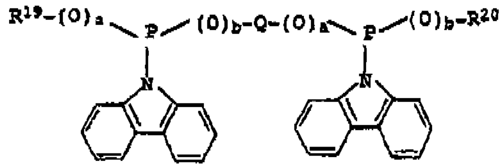

(II.2)

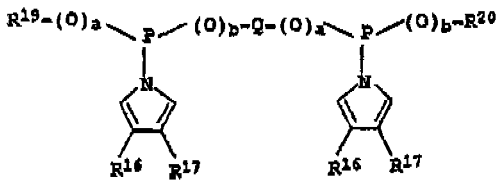

(II.3)

dans lesquelles

$R^{15}$, $R^{16}$, $R^{17}$, $R^{18}$, Q a et b ont les significations indiquées dans la revendication 4, au moins l'un des radicaux $R^{16}$ et $R^{17}$ dans la formule II.3 ne représentant pas un atome d'hydrogène,
$R^{19}$ et $R^{20}$ représentent, indépendamment l'un de l'autre, un groupe cycloalkyle, hétérocycloalkyle, aryle ou hétéroaryle.

**6.** Procédé selon l'une quelconque des revendications 1 à 5, dans lequel le groupe pontant Q représente un groupe triptycènediyle de formule

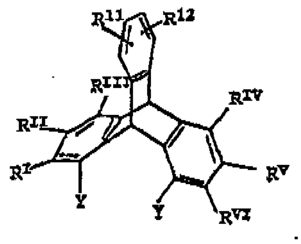

ou de formule

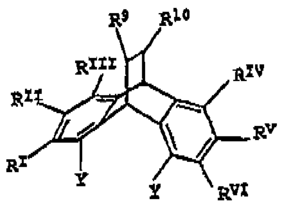

dans lesquelles $R^I$, $R^{II}$, $R^{III}$, $R^{IV}$, $R^V$ et $R^{VI}$, $R^9$, $R^{10}$, $R^{11}$ et $R^{12}$ ont les significations données dans la revendication 1.

**7.** Procédé selon l'une quelconque des revendications 1 à 5, **caractérisé en ce que** le groupe pontant Q représente un groupe xanthènediyle de formule

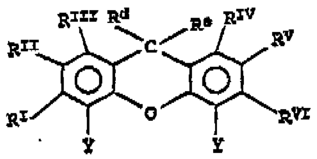

dans laquelle $R^I$, $R^{II}$, $R^{III}$, $R^{IV}$, $R^V$ et $R^{VI}$ et Y ont les significations données dans la revendication 1 et $R^d$ et $R^e$ représentent, indépendamment l'un de l'autre, un atome d'hydrogène ou un groupe alkyle, cycloalkyle, hétérocycloalkyle, aryle ou hétéroaryle.

**8.** Procédé selon l'une quelconque des revendications précédentes, dans lequel on ajuste dans le mélange réactionnel un rapport molaire du ligand au métal du groupe VIIIB de 1:1 à 1000:1.

**9.** Procédé selon l'une quelconque des revendications précédentes, dans lequel on effectue la réaction à des températures dans la plage de 40 à 80 °C.

**10.** Procédé selon l'une quelconque des revendications précédentes, **caractérisé en ce que**

(i) le 1,9-décadiène est soumis à la réaction d'hydroformylation dans une zone de réaction,
(ii) une quantité est déchargée de la zone de réaction et scindée en une fraction enrichie en monoaldéhydes insaturés et une fraction appauvrie en monoaldéhydes insaturés, et
(iii) la fraction appauvrie en monoaldéhydes insaturés, éventuellement traitée, est renvoyée dans la zone de réaction.

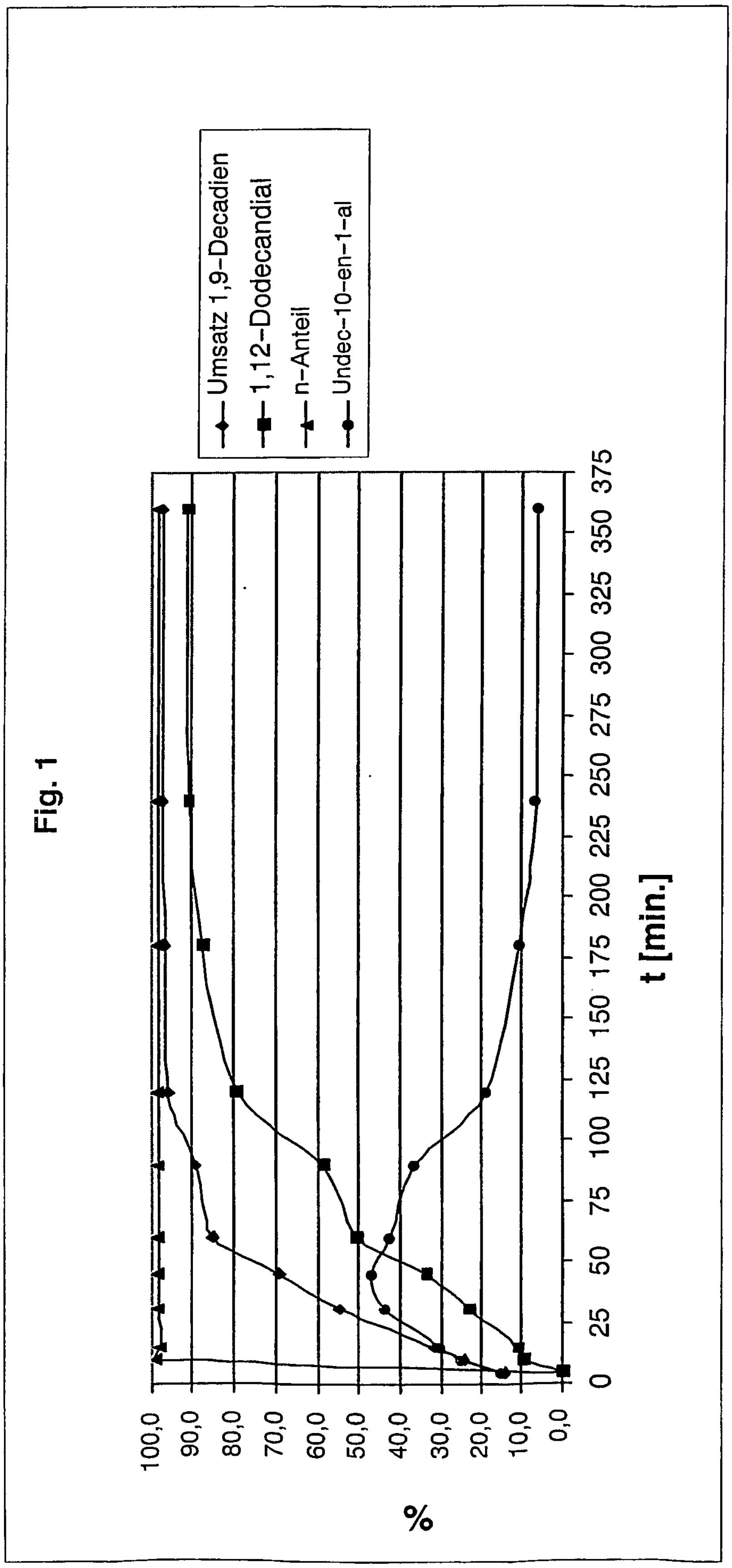
Umsatz 1,9-Decadien
1,12-Dodecandial
n-Anteil
Undec-10-en-1-al
%
100,0
90,0
80,0
70,0
60,0
50,0
40,0
30,0
20,0
10,0
0,0
0
25
50
75
100
125
150
175
200
225
250
275
300
325
350
375
t [min.]

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9530680 A
- WO 0056451 A
- WO 0158589 A
- DE 10023471 A
- DE 10046026 A
- US 5710344 A
- WO 03018192 A
- EP 0301245 W
- WO 02083695 A


### In der Beschreibung aufgeführte Nicht-Patentliteratur


- **VAN LEEUWEN et al.** *Organometallics,* 1995, vol. 14, 3081
- **VAN DER SLOT et al.** *Organometallics,* 2000, vol. 19, 2504
- **A. M. TRZECIAK ; J. J. ZIOLKOWSKI.** *J. Organomet. Chem.,* 1994, vol. 464, 107-111
- **C. BOTTEGHI et al.** *J. Mol. Catal. A: Chem,* 2001, vol. 175, 17-25
- Ullmanns Encyklopädie der technischen Chemie. 1951, vol. 1, 743 ff
- Ullmanns Encyklopädie der technischen Chemie. 1951, vol. 1, 769 ff
- **C. BOTTEGHI et al.** *J. Mol. Catal. A: Chem,* 2001, vol. 175, 17